(19) Europäisches Patentamt
European Patent Office
Office européen des brevets

(11) Numéro de publication: 0 230 844 B1

(12) # FASCICULE DE BREVET EUROPEEN

(45) Date de publication du fascicule du brevet: **11.07.90**

(51) Int. Cl.[5]: **C07D 405/06**, C07D 307/20, A01N 43/653

(21) Numéro de dépôt: **86420304.7**

(22) Date de dépôt: **18.12.86**

(54) **Fongicides à groupes triazole et oligoéther et associations.**

(30) Priorité: **20.12.85 FR 8519194**
**11.04.86 FR 8605411**
**19.06.86 FR 8609056**

(43) Date de publication de la demande: **05.08.87 Bulletin 87/32**

(45) Mention de la délivrance du brevet: **11.07.90 Bulletin 90/28**

(84) Etats contractants désignés: **AT BE CH DE ES FR GB GR IT LI LU NL SE**

(56) Documents cités:
**EP-A- 0 121 979**
**EP-A- 0 151 084**

(73) Titulaire: **RHONE-POULENC AGROCHIMIE, 14-20, rue Pierre Baizet, F-69009 Lyon(FR)**

(72) Inventeur: **Greiner, Alfred, 31 Chemin des Aulndes, F-69570 Dardilly(FR)**
Inventeur: **Souche, Jean Luc, 47 Chemin de Fontanières, F-69350 La Mulatiere(FR)**
Inventeur: **Merindoi, Béatrice, 4 Clos des Coquilles, F-69340 Francheville Le Haut(FR)**

(74) Mandataire: **Ranguis, Patrick Frédéric et al, RHONE POULENC AGROCHIMIE 14-20 Rue Pierre Baizet, F-69009 Lyon(FR)**

EP 0 230 844 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**Description**

La présente invention concerne un nouveau composé, à usage phytosanitaire, à groupements triazole et oligoéther et les associations de ce produit avec certains fongicides. L'invention concerne également des procédés de préparation dudit composé ainsi que l'application pour la protection des végétaux, spécialement dans le cadre de la lutte contre les champignons parasites mais aussi de la régulation de croissance des végétaux dudit composé et desdites associations.

De nombreux composés à groupe triazole, notamment des fongicides, sont déjà connus en particulier par la demande de brevet européen n°151084.

Un but de l'invention est de fournir des composés et associations ayant une activité et/ou une sélectivité améliorées.

Un autre but de l'invention est de fournir des composés et associations utilisables non seulement contre les attaques fongiques des céréales mais aussi contre les attaques fongiques de la vigne, des cultures maraîchères et plus particulièrement de l'arboriculture et contre les attaques des rouilles des céréales.

Un autre but de l'invention est de fournir des composés et associations présentant des risques toxicologiques amoindris par rapport à certains produits connus.

Il a maintenant été trouvé que ces buts pouvaient être atteints d'une part grâce au composé de l'invention qui a pour formule

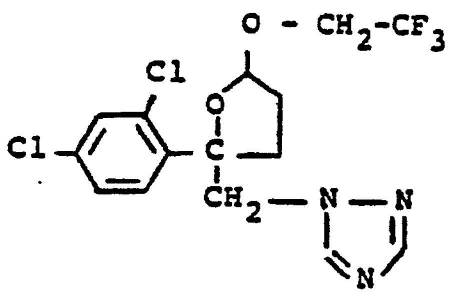

(I)

ainsi que les sels acceptables en agriculture de ce composé.

Le composé selon l'invention peut exister sous deux formes de diastéréoisomères. L'invention concerne donc aussi bien un mélange des deux formes diastéréo isomériques que les deux diastéréoisomères pris séparément. La séparation des diastéréoisomères peut s'effectuer selon les méthodes connues en soi.

L'invention concerne également les formes salifiées du composé selon l'invention et plus spécialement les chlorhydrate, sulfate, oxalate, nitrate ou arylsulfonates ainsi que les complexes d'addition de ce composé avec des sels métalliques, et notamment des sels de fer, chrome, cuivre, manganèse, zinc, cobalt, étain, magnésium et aluminium. L'ensemble des produits constitués par le produit de formule (I), ses diastéréisomères et ses dérivés sont désignés ci-après par l'expression, au pluriel: les composés selon l'invention.

Il a également été trouvé que les buts précités pouvaient être atteints grâce à l'association d'un ou plusieurs composés de formule (I) avec au moins un fongicide du groupe (II) c'est-à-dire un fongicide choisi dans les sous-classes suivantes :

1. Les dérivés chlorés ou nitrés du benzène comme le quintozène ou le chlorothalonil,
2. Les dérivés dicarboximides comme le captane, le folpel, le captafol, l'iprodione, la procymidone,
3. Les dérivés comprenant un ou plusieurs hétérocycles comme les quinoléines (éthoxyquine), les morpholines (dodémorphe, tridémorphe, fenpropimorphe), les pipéridines (fenpropidine),
4. Les dérivés de l'acide phosphoreux comme les phosphites métalliques tels que le phoséthyl-Al et l'acide phosphoreux lui-même,
5. Les dérivés de l'acide dithiocarbamique comme le manèbe, le mancozèbe ou le zinèbe,
6. Les dérivés du phénol comme le dinocap ou le binapacryl,
7. Les dérivés des quinones comme le dithianon, le chloranil,
8. Les dérivés de l'acide carbamique et des benzimidazoles comme le carbendazime, le bénomyl, le thiophanate-méthyl,
9. Les dérivé soufrés comme le dazomet ou l'étridiazole ou le soufre,
10. Les amines et les amides telles que le dichloran, la carboxine, la triforine, le cymoxanil, le métalaxyl, l'ofurace,
11. Les diazines telles que le chinométhionate, le fénarimol, l'anilazine, le nuarimol, le bupirimat, l'éthylrimol, le pyrazophos,
12. Les sulfamides telles que le dichlofluanide, le tolylfluanide,
13. Les guanidines telles que la doguadine,

14. Les triazoles tels que le diniconazole, le propiconazole, le triadimephon, le triadimenol, le diclobutrazol, le bitertanol, le penconazol, le flutriafol,
15. Les imidazoles comme le prochloraz ou l'imazalil,
16. Le cuivre ou les dérivés organiques ou inorganiques du cuivre.

Les matières actives du groupe (II) sont des matières actives connues, la plupart d'entre elles étant décrites en détail dans des ouvrages tels que "The Pesticide Manual" édité par "The British Crop Protection Council" dont la 7ème édition date de 1983.

Les associations selon l'invention sont le plus souvent de type binaire (une seule matière active du groupe II) mais on utilise aussi quelquefois des associations ternaires (2 matières actives du groupe II) ou quaternaire (3 matières actives du groupe II).

Parmi les matières actives, on préfèrera celles choisies parmi les sous-classes 1, 2, 3, 4, 5, 6, 7, 8, 9, 16.

Parmi les matières actives du groupe II, on préfèrera encore les matières actives suivantes : chlorothalonil, iprodione, fenpropimorphe, tridémorphe, fenpropidine, dinocap, dithianon, manèbe, mancozèbe, zinèbe, phoséthyl-Al, cymoxanil, nuarimol, captane, carbendazime captafol, soufre, le cuivre et les dérivés du cuivre tels que l'oxychlorure de cuivre ou quinoléate de cuivre.

Les noms chimiques des produits énumérés ci-dessus sont indiqués au tableau annexé selon la nomenclature anglo-saxonne, c'est-à-dire avec l'indication de la position des substituants placée avant celui-ci.

Le rapport pondéral du composé selon l'invention avec les matières actives du groupe II décrite cidessus est de préférence compris entre 0,0003 et 3000 et avantageusement entre 0,001 et 1000.

La présente invention concerne également des procédés de préparation des composés selon l'invention ; plusieurs de ces procédés sont décrits dans la demande de brevet européen n°151084 dont la substance est incorporée ici par référence.

Selon un premier procédé, on fait réagir un composé de formule :

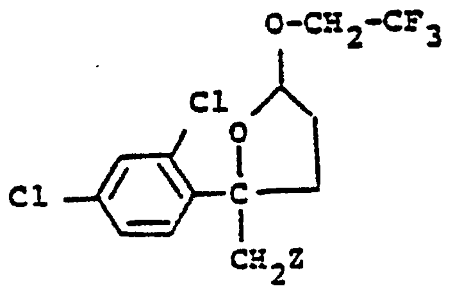

(II)

dans laquelle Z est un atome de chlore ou de brome, avec un dérivé alcalin (par exemple un sel de sodium ou de potassium), ou un dérivé d'ammonium ou phosphonium quaternaire) du triazole.

La réaction s'effectue habituellement en milieu solvant polaire aprotique et peut aussi être catalysée, par exemple par addition d'iodure alcalin ; la température est généralement comprise entre 50 et 250°C, de préférence entre 70 et 230°C. Pour des raisons économiques, les concentrations globales en réactifs comprises entre 1 et 50% sont le plus souvent utilisées.

Les composés de formule (II) peuvent se préparer par réaction de l'alcool $CF_3$-$CH_2OH$ (trifluoroéthanol) avec un composé de formule

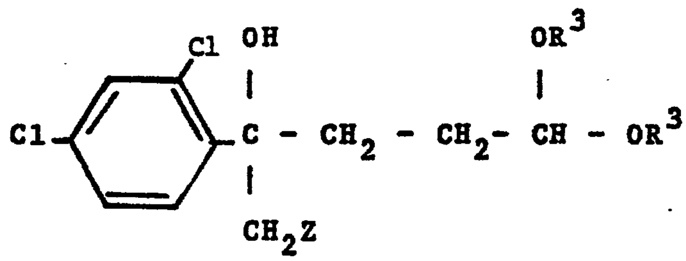

(III)

en présence d'un catalyseur acide, Z ayant la même signification que précédemment et $R^3$ étant un radical organique, de préférence alkyle inférieur ($C_{1-4}$), deux radicaux $R^3$ pouvant constituer ensemble un radical organique divalent, de préférence alkylène inférieur.

L'acide catalyseur mis en oeuvre dans cette réaction peut être un acide protique ou aprotique. Comme acides protiques, on peut citer les acides chlorhydrique, sulfurique, trifluoroacétique, perchlorique,

benzène-sulfonique, toluène-sulfonique, méthane-sulfonique. Comme acides aprotiques on peut citer les acides de Lewis tels que $BF_3$, $AlCl_3$ et $SnCl_4$. Lorsqu'on utilise, comme catalyseur, l'acide chlorhydrique, ce dernier peut être généré in situ, par exemple à l'aide de chlorure d'acyle, et notamment de chlorure d'acétyle, lequel réagit sur l'alcool présent pour donner naissance à HCl.

La réaction s'effectue ordinairement par simple chauffage des réactifs indiqués. La température est généralement comprise dans la zone de température allant de 50°C à la température d'ébullition du milieu réactionnel. Le trifluoroéthanol joue habituellement le rôle de solvant dans le milieu réactionnel. Un cosolvant inerte peut être également ajouté, notamment un hydrocarbure aliphatique, alicyclique ou aromatique, halogéné ou non, ou un éther.

Les composés de formule (III) se préparent selon des procédés connus en soi.

Selon un autre procédé de préparation de composés selon l'invention, on fait réagir le trifluoroéthanol ($CF_3$-$CH_2OH$) avec un composé de formule

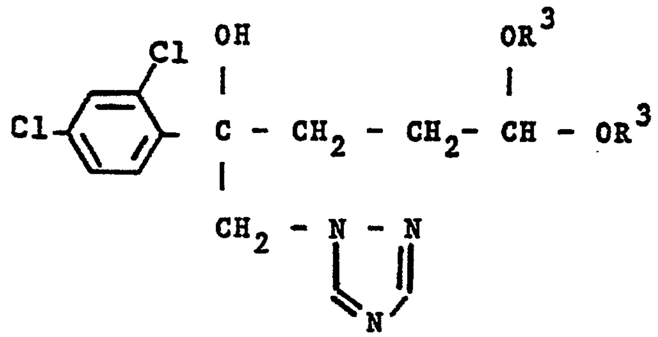

(IV)

en présence d'un catalyseur acide. Les divers symboles indiqués pour les formules de ces deux réactifs ont les significations données plus haut. Comme catalyseur acide on peut également utiliser ceux définis plus haut à propos de la préparation des composés de formule (II). Les autres conditions réactionnelles sont aussi semblables à celles définies pour la préparation de ces composés de formule (IIa).

Les composés de formule (IV) peuvent s'obtenir selon des procédés connus en soi.

Les associations sont préparées par simple mélange des produits et composés souhaités.

La présente invention concerne également les composés de formule (II) dans laquelle Z est l'atome de chlore ou de brome. Ces composés sont éventuellement utilisables comme intermédiaires pour la préparation des composés de formule (I).

La présente invention concerne également l'utilisation des composés de formule (I) et des associations précédemment définies à titre de fongicide.

Les composés et les associations selon l'invention peuvent donc être utilisés pour la lutte tant préventive que curative contre les champignons, notamment de type basidiomycètes, ascomycètes, adelomycètes ou fungi-imperfecti, en particulier les rouilles, oïdium, piétin-verse, fusarioses, helminthosporioses, septorioses, rhizoctones des végétaux et des plantes en général et en paraticulier des céréales telles que le blé, l'orge, le seigle, l'avoine et leurs hybrides et aussi le riz et le maïs.

Les composés selon l'invention sont actifs en particulier contre les champignons mentionnés dans la demande européenne 151084. Ils sont aussi et encore actifs contre les champignons suivants : Acrostalagmus koningi, les Alternaria, les Colletotrichum, Corticium rolfsii, Diplodia natalensis, Gaeumannomyces graminis, Gibberella fujikuroi, Hormodendron cladosporioides, Lentinus degener ou tigrinus, Lenzites quercina, Memnoniella echinata, Myrothecium verrucaria, Paecylomyces varioti, Pellicularia sasakii, Phellinus megaloporus, Polystictus sanguineus, Poria vaporaria, Sclerotium rolfsii, Stachybotris atra, les Stereum, Stilbum sp. Trametes trabea, Trichoderma pseudokoningi, Trichothecium roseum.

Les composés et les associations de l'invention sont spécialement intéressants par leur spectre large au niveau des maladies des céréales (oïdium, rouille, piétin-verse, helminthosporioses, septorioses et en paraticulier les fusarioses difficiles à combattre). Ils présentent également un grand intérêt en raison de leur activité sur la pourriture grise (Botrytis) et les cercosporioses, et, de ce fait, ils peuvent être appliqués sur des cultures aussi variées que la vigne, les cultures maraîchères et l'arboriculture et les cultures tropicales telles que l'arachide, le bananier, le caféier, le palmier, le cocotier, la noix de pécan et d'autres.

Ils présentent enfin une excellente sélectivité vis-à-vis des cultures.

Outre les applications déjà décrites plus haut, les produits selon l'invention présentent en outre une excellente activité biocide à l'égard de nombreuses autres variétés de microorganismes parmi lesquelles on peut citer à titre non limitatif, des champignons comme ceux des genres :

- Pullularia comme l'espèce P. Pullulans,
- Chaeotomium comme l'espèce C. Globosum,
- Aspergillus comme l'espèce Aspergillus niger,

- Coniophora comme l'espèce C. Puteana.

En raison de leur activité biocide, les produits de l'invention permettent de combattre efficacement les microorganismes dont la prolifération crée de nombreux problèmes dans les domaines agricole et industriel. A cet effet, ils conviennent tout spécialement bien à la protection des végétaux ou de produits industriels tels que le bois, le cuir, les peintures, le papier, les cordages, les plastiques, les circuits d'eau industriels.

Ils sont tout particulièrement bien adaptés à la protection des produits lignocellulosiques et notamment du bois, qu'il s'agisse de bois d'ameublement, de charpente ou de bois exposé aux intempéries tels que les bois de clôture, les piquets de vignes, les traverses de chemin de fer.

Les composés ou associations selon l'invention utlisés, seuls ou sous la forme de compositions telles que définies ci-dessus, dans les traitements du bois peuvent être éventuellement associés à un ou plusieurs produits biocides connus tels que le pentachlorophénol, les sels métalliques, notamment de cuivre, de manganèse, de cobalt, de chrome, de zinc dérivés d'acides minéraux ou carboxyliques (acides heptanoïque, octanoïque, naphténique); les complexes organiques de l'étain, le mercaptobenzothiazole.

Ils s'appliquent généralement à des doses de 0,005 à 5 kg/ha, avantageusement à des doses de 0,01 à 5kg/ha, de préférence de 0,05, plus spécifiquement de 0,1, à 2kg/ha.

L'utilisation d'au moins un des composés de formule (I) en association avec l'iprodione est particulièrement intéressante dans le traitement des maladies des céréales, des semences comme des maladies foliaires.

L'utilisation d'au moins un des composés de formule (I) en association avec la fenpropidine est particulièrement intéressante dans le traitement des maladies des céréales, des semences comme des maladies foliaires.

L'utilisation d'au moins un des composés de formule (I) en association avec le fenpropimorphe est partaiculièrement intéressante dans le traitement des maladies des céréales et des betteraves.

L'utilisation d'au moins un des composés de formule (I) en association avec le tridemorphe est particulièrement intéressante dans le traitement des maladies des céréales.

Bien entendu il est possible d'utiliser des mélanges ternaires ou quaternaires avec les composés cités ci-dessus pour les traitements indiqués.

Ainsi l'utilisation d'un mélange ternaire à base de fenpropimorphe, d'iprodione et d'un composé selon l'invention ou de tridémorphe, d'iprodione et d'un composé selon l'invention est particulièrement intéréssante.

L'utilisation d'au moins un des composés de formule (I) en association avec le manèbe est particulièrement intéressante dans le traitement des maladies des cultures légumières, des arbres fruitiers et fruits et des vignes.

L'utilisation d'au moins un des composés de formule (I) en association avec le mancozèbe est particulièrement intéressante dans le traitement des maladies des cultures légumières, des arbres fruitiers et fruits et des vignes.

L'utilisation d'au moins un des composés de formule (I) en association avec le zinèbe est particulièrement intéressante dans le traitement des maladies des cultures légumières, des arbres fruitiers et fruits et des vignes.

L'utilisation d'au moins un des composés de formule (I) en association avec le soufre est particulièrement intéressante dans le traitement des maladies des cultures légumières, des arbres fruitiers et fruits et des vignes.

L'utilisation d'au moins un des composés de formule (I) en association avec le dinocap est particulièrement intéressante dans le traitement des maladies des cultures légumières, des arbres fruitiers et fruits et des vignes.

Bien entendu il est possible d'utiliser des mélanges ternaires ou quaternaires avec les composés cités ci-dessus pour les traitements indiqués.

L'utilisation d'au moins un des composés de formule (I) en association avec le carbendazime est particulièrement intéressante dans le traitement des maladies des arbres fruitiers et fruits, des cultures légumières, des céréales.

L'utilisation d'au moins un des composés de formule (I) en association avec le captafol est particulièrement intéressante dans le traitement des maladies des céréales, des arbres fruitiers et fruits, de la pomme de terre.

L'utilisation d'au moins un des composés de formule (I) en association avec le captane est particulièrement intéressante dans le traitement des maladies des céréales, des arbres fruitiers et fruits.

L'utilisation d'au moins un des composés de formule (I) en association avec le dithianon est particulièrement intéressante dans le traitement des maladies des arbres fruitiers et fruits.

L'utilisation d'au moins un des composés de formule (I) en association avec le mancozèbe est particulièrement intéressante dans le traitement des maladies des arbres fruitiers et fruits.

L'utilisation d'au moins un des composés de formule (I) en association avec le cuivre est particulièrement intéressante dans le traitement des maladies des arbres fruitiers et fruits.

Bien entendu il est possible d'utiliser des mélanges ternaires ou quaternaires avec les composés cités ci-dessus pour les traitements indiqués.

L'utilisation d'au moins un des composés de formule (I) en association avec le chlorotalonil est particulièrement intéressante dans le traitement des maladies des cultures légumières, des céréales, des betteraves, de la pomme de terre.

L'utilisation d'au moins un des composés de formule (I) en association avec le diniconazole est particulièrement intéressante dans le traitement des charbons des céréales, en particulier les charbons nus de l'orge et du blé (ustilago nuda).

Pour leur emploi pratique, les composés et les associations selon l'invention sont rarement utilisés seuls. Le plus souvent ils font partie de compositions. Ces compositions, utilisables pour la protection des végétaux contre les maladies fongiques, ou dans les compositions régulatrices de la croissance des plantes, contiennent comme matière active un composé selon l'invention tel que décrit précédemment en association avec les supports solides ou liquides, acceptables en agriculture et/ou les agents tensio-actifs également acceptables en agriculture. En particulier sont utilisables les supports inertes et usuels et les agents tensio-actifs usuels.

Par le terme "support", dans le présent exposé, on désigne une matière organique ou minérale, naturelle ou synthétique, avec laquelle la matière active est associée pour faciliter son application sur la plante, sur des graines ou sur le sol. Ce support est donc généralement inerte et il doit être acceptable en agriculture, notamment sur la plante traitée. Le support peut être solide (argiles, silicates naturels ou synthétiques, silice, résines, cires, engrais solides, etc...) ou liquide (eau, alcools, cétones, fractions de pétrole, hydrocarbures aromatiques ou paraffiniques, hydrocarbures chlorés, gaz liquéfiés, etc...).

L'agent tensioactif peut être un agent émulsionnant, dispersant ou mouillant de type ionique ou non ionique. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés. La présence d'au moins un agent tensioactif est généralement indispensable lorsque la matière active et/ou le support inerte ne sont pas solubles dans l'eau et que l'agent vecteur de l'application est l'eau.

Pour leur application, les composés de formule (I) et les associations se trouvent donc généralement sous forme de compositions ; ces compositions selon l'invention sont elles-mêmes sous des formes assez diverses, solides ou liquides.

Comme formes de compositions solides, on peut citer les poudres pour poudrage ou dispersion (à teneur en composé de formule (I) pouvant aller jusqu'à 100 %) et les granulés, notamment ceux obtenus par extrusion, par compactage, par imprégnation d'un support granulé, par granulation à partir d'une poudre (la teneur en composé de formule (I) dans ces granulés étant entre 1 et 80 % pour ces derniers cas).

Comme formes de compositions liquides ou destinées à constituer des compositions liquides lors de l'application, on peut citer les solutions, en particulier les concentrés solubles dans l'eau, les concentrés émulsionnables, les émulsions, les suspensions concentrées, les aérosols, les poudres mouillables (ou poudre à pulvériser), les pâtes.

Ces compositions peuvent contenir aussi toute sorte d'autres ingrédients tels que, par exemple, des colloïdes protecteurs, des adhésifs, des épaississants, des agents thixotropes, des agents de pénétration, des stabilisants, des séquestrants, etc... ainsi que d'autres matières actives connues à propriétés pesticides (notamment insecticides ou fongicides) ou à propriétés favorisant la croissance des plantes (notamment des engrais) ou à propriétés régulatrices de la croissance des plantes. Plus généralement les composés selon l'invention peuvent être associés à tous les additifs solides ou liquides correspondant aux techniques habituelles de la mise en formulation.

Ces doses d'emploi dans le cas d'une utilisation comme fongicides des composés et des associations selon l'invention peuvent varier dans de larges limites, notamment selon la virulence des champignons et les conditions climatiques.

D'une manière générale des compositions contenant 0,5 à 5000 ppm de substance active conviennent bien ; ces valeurs sont indiquées pour les compositions prêtes à l'application. Ppm signifie "partie par million". La zone de 0,5 à 5000 ppm correspond à une zone de $5x10^{-5}$ à 0,5 % (pourcentages pondéraux).

En ce qui concerne les compositions adaptées au stockage et au transport, elles contiennent plus avantageusement de 0,5 à 95 % (en poids) de substance active.

Ainsi donc, les compositions à usage agricole selon l'invention peuvent contenir les matières actives selon l'invention dans de très larges limites, allant de $5.10^{-5}$% à 95 % (en poids).

D'une façon générale, les compositions selon l'invention contiennent habituellement de 0,05 à 95 % environ de matières actives (formule I et groupe II) selon l'invention, de 1 % à 95 % environ de un ou plusieurs supports solides ou liquides et éventuellement de 0,1 à 50 %, de préférence 5 à 40 %, environ de un ou plusieurs agents tensioactifs.

Les concentrés émulsionnables ou solubles comprennent le plus souvent 10 à 80 % de matière active, les émulsions ou solutions prêtes à l'application contenant, quant à elles, 0,01 à 20 % de matière active. En plus du solvant, les concentrés émulsionnables peuvent contenir, quand c'est nécessaire, 2 à 20 % d'additifs appropriés, comme les stabilisants, agents tensioactifs, agents de pénétration, inhibiteurs de corrosion, colorants, adhésifs précédemment cités.

A titre d'exemple, voici la composition de quelques concentrés :

Exemple F (formulation) 1

- matière active 400 g/l
- dodécylbenzène sulfonate alcalin 24 g/l
- nonylph'enol oxyéthylé à 10 molécules d'oxyde d'éthylène 16 g/l
- cyclohexanone 200 g/l
- solvant aromatique q.s.p. 1 litre

Selon une autre formule de concentré émulsionnable, on utilise :

Exemple F 2:

- matière active 250 g
- huile végétale époxydée 25 g
- mélange de sulfonate d'alcoylaryle et d'éther de polyglycol et d'alcools gras 100 g
- diméthylformamide 50 g
- xylène 575 g

A partir de ces concentrés, on peut obtenir par dilution avec de l'eau des émulsions de toute concentration désirée, qui conviennent particulièrement à l'application sur les feuilles.

Les suspensions concentrées, également applicables en pulvérisation, sont préparées de manière à obtenir un produit fluide stable ne se déposant pas et elles contiennent habituellement de 10 à 75 % de matière active, de 0,5 à 15 % d'agents tensioactifs, de 0,1 à 10 % d'agents thixotropes, de 0 à 10 % d'additifs appropriés, comme des anti-mousses, des inhibiteurs de corrosion, des stabilisants, des agents de pénétration et des adhésifs et, comme support, de l'eau ou un liquide organique dans lequel la matière active est peu ou pas soluble : certaines matières solides organiques ou des sels minéraux peuvent être dissous dans le support pour aider à empêcher la sédimentation ou comme antigels pour l'eau.

Les poudres mouillables (ou poudre à pulvériser) sont habituellement préparées de manière qu'elles contiennent 20 à 95 % de matière active, et elles contiennent habituellement, en plus du support solide, de 0 à 5 % d'un agent mouillant, de 3 à 10 % d'un agent dispersant, et, quand c'est nécessaire, de 0 à 10 % d'un ou plusieurs stabilisants et/ou autres additifs, comme des agents de pénétration, des adhésifs, ou des agents antimottants, colorants, etc...

A titre d'exemple, voici diverses compositions de poudres mouillables :

Exemple F 3:

- matière active 50 %
- lignosulfonate de calcium (défloculant) 5 %
- isopropylnaphtalène sulfonate (agent mouillant anionique) 1 %
- silice antimottante 5 %
- kaolin (charge) 39 %

Une autre composition de poudre à pulvériser à 70 % utilise les constituants suivants :

Exemple F 4:

- matière active 700 g
- dibutylnaphtylsulfonate de sodium 50 g
- produit de condensation en proportions 3/2/1 d'acide naphtalène sulfonique, d'acide phénolsulfonique et de formaldéhyde 30 g
- kaolin 100 g
- craie de champagne 120 g

Une autre composition de poudre à pulvériser à 40 % utilise les constituants suivants :

Exemple F 5 :

- matière active 400 g
- lignosulfonate de sodium 50 g
- dibutylnaphtalène sulfonate de sodium 10 g
- silice 540 g

Une autre composition de poudre à pulvériser à 25 % utilise les constituants suivants :

Exemple F 6 :

-matière active 250 g
- lignosulfonate de calcium 45 g
- mélange équipondéral de craie de Champagne et d'hydroxyéthylcellulose 19 g
- dibutylnaphtalène sulfonate de sodium 15 g
- silice 195 g
- craie de Champagne 195 g
- kaolin 281 g

Une autre composition de poudre à pulvériser à 25 % utilise les constituants suivants :

Exemple F 7 :

- matière active 250 g
- issoctylphénoxy-polyoxyéthylène-éthanol 25 g
- mélange équipondéral de craie de Champagne et d'hydroxyéthylcellulose 17 g
- aluminosilicate de sodium 543 g
- kieselguhr 165 g

Une autre composition de poudre à pulvériser à 10 % utilise les constituants suivants :

Exemple F 8 :

- matière active 100 g
- mélange de sels de sodium de sulfates d'acides gras saturés 30 g
- produit de condensation d'acide naphtalène sulfonique et de formaldéhyde 50 g
- kaolin 820 g

Pour obtenir ces poudres à pulvériser ou poudres mouillables, on mélange intimement les matières actives dans des mélangeurs appropriés avec les substances additionnelles et on broie avec des moulins ou autres broyeurs appropriés. On obtient par là des poudres à pulvériser dont la mouillabilité et la mise en suspension sont avantageuses ; on peut les mettre en suspension avec de l'eau à toute concentration désirée et ces suspensions sont utilisables très avantageusement en particulier pour l'application sur les feuilles des végétaux.

A la place des poudres mouillables, on peut réaliser des pâtes. Les conditions et modalités de réalisation et d'utilisation de ces pâtes sont semblables à celles des poudres mouillables ou poudres à pulvériser.

Comme cela a déjà été dit, les dispersions et émulsions aqueuses, par exemple les compositions obtenues en diluant à l'aide d'eau une poudre mouillable ou un concentré émulsionnable selon l'invention, sont comprises dans le cadre général de la présente invention. Les émulsions peuvent être du type eau-dans-l'huile ou huile-dans-l'eau et elles peuvent avoir une consistance épaisse comme celle d'une "mayonnaise".

Les granulés destinés à être disposés sur le sol sont habituellement préparés de manière qu'ils aient des dimensions comprises entre 0,1 et 2 mm et ils peuvent être fabriqués par agglomération ou imprégnation. En général, les granulés contiennent 0,5 à 25 % de matière active et 0 à 10 % d'additifs comme des stabilisants, des agents de modification à libération lente, des liants et des solvants.

Selon un exemple de composition de granulé, on utilise les constituants suivants :

Exemple F 9 :

- matière active 50 g
- épichlorhydrine 2,5 g
- éther de cétyle et de polyglycol 2,5 g
- polyéthylène glycol 35 g
- kaolin (granulométrie : 0,3 à 0,8 mm) 910 g

Dans ce cas particulier on mélange la matière active avec l'épichlorhydrine et on dissout avec 60 g d'acétone ; on ajoute alors le polyéthylène glycol et l'éther de cétyle et de polyglycol. On arrose le kaolin avec la solution obtenue et on évapore ensuite l'acétone sous vide. On utilise avantageusement un tel microgranulé pour lutter contre les champignons du sol.

Les composés de formule (I) et les associations peuvent encore être utilisés sous forme de poudres pour poudrage ; on peut aussi utiliser une composition comprenant 50 g de matière active et 950 g de

talc; on peut aussi utiliser une composition comprenant 20 g de matière active, 10 g de silice finement divisée et 970 g de talc ; on mélange et broie ces constituants et on applique le mélange par poudrage.

Les exemples suivants donnés à titre non limitatif illustrent l'invention et montrent comment elle peut être mise en oeuvre.

Les exemples 1 à 3 illustrent des modes particuliers de préparation de composés selon l'invention, ainsi que ces composés eux-mêmes. Parmi les propriétés physiques indiquées pour ces composés, on a indiqué les valeurs des déplacements (delta) en RMN du proton du groupe -O-CH-O- (acétal). Ces déplacements sont mesurés en ppm et ils sont repérés par rapport à un produit de référence qui et le tétraméthyl silane. La RMN est effectuée à 100 MHz dans le chloroforme deutéré.

Les exemples 4 à 10 illustrent les propriétés fongicides des composés selon l'invention ainsi que leurs applications.

Exemple 1 :

On prépare un produit de formule :

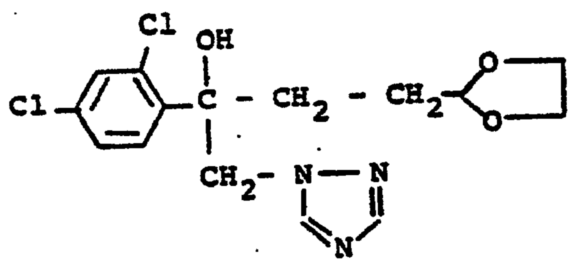

(V)

Selon un procédé décrit dans la demande de brevet européen n°151084, on mélange 40 ml de trifluoroéthanol avec 10,7 g de produit de formule (V). On fait barboter du HCl gazeux de manière à avoir une augmentation pondérale de 1,39 g. On chauffe 2 heures à 70°C. On verse dans 500 ml d'eau contenant 10 g de $Na_2CO_3$. On extrait à l'acétate d'éthyle ; la solution organique est lavée à l'eau, sèchée, filtrée, évaporée. On obtient ainsi 8 g de cristaux constitués d'un mélange de 2 diastéréoisomères du produit de formule (I), fondant à prtir de 80°C et présentant en RMN un déplacement protonique delta à 5,18 et 5,41 ppm

Exemple 2 :

Le produit obtenu à l'exemple 1 est traité en chromatographie liquide sur colonne de silice sous pression de 0,3 bar (au dessus de la pression atmosphérique) avec un éluant constitué d'un mélange acétate d'éthyle/heptane en proportions volumiques 80/20.

On recueille 3 g d'une première fraction A et 3,6 g d'une deuxième fraction B.

Chacune de ces fractions donne un précipité qui cristallise. Les cristaux A issus de la fraction A correspondent à l'un des diastéréoisomères. Ils fondent à 84°C et ont un déplacement protonique delta en RMN à 5,41 ppm. Le diastéréoisomère A est caractérisé par le fait que le groupe triazolylméthyle et le groupement trifluoroéthoxy sont situés d'un même côté du plan du cycle tétrahydrofurannique.

Les cristaux B issus de la fraction B correspondent à l'autre diastéréoisomère. Ils fondent à 167°C et ont un déplacement protonique delta en RMN à 5,18 ppm.

Exemple 3 :

On mélange 40 ml de trifluoroéthanol et 10,8 g de produit de formule

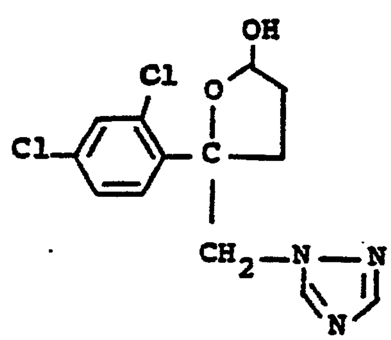

préparé comme indiqué dans la demande de brevet européen n°151084.

On fait barboter du HCl gazeux jusqu'à une augmentation de poids de 1,5 g. On chauffe 4 h à 70°C, verse dans 500 ml d'eau contenant 10 g de $Na_2CO_3$. On extrait à l'aide de $CHCl_3$ ; la phase organique est lavée à l'eau, sèchée, évaporée. On obtient 12,7 g de produit de formule (I) sous forme de mélange des 2 diastéréoisomères.

Exemple 4 :

On a traité 4 parcelles de terrain portant chacune 4 pommiers de la variété connue sous le nom "Calville" qui est très sensible à la tavelure (maladie dont le nom latin est Venturia inaequalis et le nom anglais est scab). Les traitements intervenaient tous les 12 jours depuis la floraison jusqu'au stade de milieu de grossissement des fruits. Ces traitements constituaient à appliquer une bouillie c'est à dire une émulsion aqueuse diluée de la matière active décrite à l'exemple 1. Cette bouillie a été appliquée à raison de 10 hl/ha et a été obtenue par dilution d'un concentré émulsionnable ; ce concentré contenait lui-même 62 g/l de matière active, 203 g/l de cyclohexanone, 609 g/l d'acétophénone, 50 g/l d'alkylarylsulfonate de calcium, 100 g/l d'huile de ricin polyéthoxylée ; la concentration de la bouillie en matière active était de 2,5 g/hl.

Les résultats ont été comparés avec le produit voisin de formule chimique connue la plus proche, c'est à dire la formule du composé de l'exemple 42 de la demande de brevet européen 151084 ; ce produit est dénommé ci-après produit de référence. Le produit utilisé ainsi pour la comparaison comprenait, de même que le produit de l'exemple 1 selon l'invention, la totalité des formes diastéréoisomères possibles.

On a mesuré les résultats de la manière suivante : phytotoxicité : mesurée un jour après le 3ème traitement et exprimée en pourcentage selon une appréciation visuelle de l'état général des feuilles et fruits ; la valeur de 15 % est la valeur maximum acceptable,activité feuille : mesurée un jour après le 5ème traitement et exprimée en pourcentage du nombre de feuilles atteintes de tavelure, activité fruits : mesurée en fin de traitement et exprimée en pourcentage du nombre de fruits atteints.

Les résultats ont été les suivants :

| | phytotoxicité | activité feuilles | activité fruits |
|---|---|---|---|
| produit de l'ex.1 | 1 | 32,2 | 4,4 |
| produit de référence | 1 | 80,0 | 58,7 |
| témoin non traité | 0 | 99,8 | 84,2 |

Exemple 5 :

On opère comme à l'exemple 4, mais en traitant de pommiers de la variété Golden avec une bouillie ayant une concentration de matière active de 5 g/hl.

Les résultats sont les suivants :

| | phytotoxicité | activité feuilles | activité fruits |
|---|---|---|---|
| produit de l'ex.1 | 0 | 3 | 0 |
| produit de référence | 0 | 39,8 | 5,9 |
| témoin non traité | 0 | 99,7 | 87 |

Exemple 6 :

On a traité 4 parcelles de terrain portant chacune 15 ceps de vigne de la variété connue sous le nom de Carignan qui est très sensible à l'oïdium (maladie dont le nom latin est Uncinula necator et le nom anglais est powdery mildew). Les traitements avaient lieu tous les 14 jours depuis l'éclatement des bourgeons jusqu'à la veraison (époque de changement de couleur des grappes de raisin).

Ces traitements constituaient à appliquer une bouillie de même nature qu'à l'exemple 4 à raison de 1,5 g/hl.

Les résultats, obtenus et comparés comme à l'exemple 4, ont été les suivants :

| | phytotoxicité (mesurée 13 j après le 2ème traitement) | activité feuille (mesurée 8 j après le 3ème traitement) | activité fruits(mesurée 14 j après le traitement) |
|---|---|---|---|
| produit de l'ex.1 | 0 | 23,5 | 0 |
| produit de référence | 0 | 29,5 | 0 |
| témoin non traité | 0 | 100 | 96,2 |

Exemple 7 à 10 :

On a traité 4 parcelles de terrain de 10 m² portant une culture de blé. Un ou deux traitements ont été effectués lors de l'apparition des premiers symptômes de maladie dans les champs.

La bouillie de traitement était de même nature qu'à l'exemple 4 et était appliquée en quantité telle que la dose de matière active était de 90, 120 ou 150 g/ha selon les essais.

Dans tous les essais, aucune phytotoxicité n'a été observée.

A l'exemple 7, on a traité une culture de blé de la variété Nebraska atteinte de rouille jaune (Puccinia striiformis), les résultats étant observés, 10 jours après le 2ème traitement, sur la feuille la plus haute juste en dessous de l'épi.

A l'exemple 8, on a traité une culture de blé de la variété Vaillant atteinte de rouille brune (Puccinia recondita), les résultats étant observés, 19 jours après le ler traitement, sur la feuille la plus haute juste en dessous de l'épi.

A l'exemple 9, on a traité une culture de blé de la variété Longbow atteinte de septoriose (Septoria tritici), les résultats étant observés, 46 jours après le traitement unique, sur la feuille la plus haute juste en dessous de l'épi.

A l'exemple 10, on a traité une culture de blé de la variété Roazon, les résultats étant observés 44 jours après le traitement unique sur la 2ème feuille depuis l'épi (la première feuille, ou feuille la plus haute étant indemne de maladie). Cette mesure tardive illustre la bonne persistance des produits.

Les résultats ont été mesurés et exprimés sous forme de :

activité feuille : pourcentage du nombre de feuilles atteintes par la maladie

activité surface de feuille : pourcentage de surface de feuille atteinte par la maladie.

Les résultats ont été les suivants :

| | dose en g/ha | exemple 7 activité feuille | exemple 8 activité feuille | exemple 8 activité surface de feuille |
|---|---|---|---|---|
| produit de l'ex.1 | 90 | 4,6 | 75 | 2,8 |
| | 120 | 2,1 | 36 | 0,8 |
| | 150 | 1,2 | 28 | 0,6 |
| produit de référence | 90 | 39,2 | 88 | 8,8 |
| | 120 | 27,3 | 93 | 5,4 |
| | 150 | 19,5 | 74 | 4,7 |
| témoin non traité | 0 | 80 | 100 | 27,2 |

| | dose en g/ha | exemple 9 activité surface de feuille | exemple 10 activité surface de feuille |
|---|---|---|---|
| produit de l'ex.1 | 90 | 3,6 | 21,6 |
| | 120 | 2,6 | 10,3 |
| | 150 | 2,9 | 3,9 |
| produit de référence | 90 | 6,9 | 10,9 |
| | 120 | 7,7 | 11,7 |
| | 150 | 4,8 | 15,9 |
| témoin non traité | 0 | 21,4 | 58,7 |

Exemple 11 :

Test in vivo sur Erysiphe graminis f.sp.hordei sur orge (oïdium de l'orge)

On prépare par broyage fin une émulsion aqueuse de la matière active à tester ayant la composition suivante :

- matière active à tester 90 mg
- Tween 80 (agent tensioactif constitué d'un oléate de dérivé polyoxyéthylèné du sorbitan) dilué à 10 % dans l'eau 0,45 ml
- eau 90 ml.

Cette émulsion aqueuse est ensuite diluée par de l'eau pour obtenir la concentration désirée.

De l'orge, en godets, semée dans de la terre franche, est traitée au stade 10 cm de hauteur par pulvérisation d'une émulsion aqueuse (appelée bouillie) de concentration indiquée ci-après. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 12 jours après la contamination.

Dans ces conditions, on observe les résultats suivants :

A la dose de 0,1 g/l, protection totale (supérieure ou égale à 95 %)avec les composés de l'exemple 1 et le composé A de l'exemple 2.

Exemple 12 :

Test in vivo sur "Puccinia recondita" responsable de la rouille brune du blé

Du blé, en godets, semé dans de la terre franche, est traité au stade 10 cm de hauteur par pulvérisation avec des émulsions aqueuses (appelées bouillies) de même composition que celle décrite à l'exemple 11 et à diverses concentrations du composé à tester. L'essai est répété deux fois avec chaque concentration.

Au bout de 24 heures, une suspension aqueuse de spores (50000 sp/$cm^3$) est pulvérisée sur le blé ; cette suspension a été obtenue à partir de plants contaminés. On place ensuite le blé pendant 48 heures en cellule d'incubation à environ 18°C et à 100 % d'humidité relative.

Au bout de ces 2 jours, l'humidité relative est ramenée à 60 %. Le contrôle de l'état des plants se fait entre le 11ème et le 15ème jour après la contamination par comparaison avec le témoin non traité.

Dans ces conditions, on observe les résultats suivants :

A la dose de 0,1 g/l, protection totale avec les composés des exemples 1 et 2 (composé A).

Exemple 13 :

Tests in vitro sur champignons des semences et champignons du sol

On étudie l'action des composés selon l'invention sur les champignons suivants responsables des maladies secondaires des céréales :

Pseudocercosporella herpotrichoïdes (CERC)
Helminthosporium gramineum (HELM G)
Botrytis cinerea (BOT)
Pyrenophorae avenae (PYRE)
Septoria nodorum (SEPT N)
Helminthosporium teres (HELM T)

Les indications figurant entre parenthèses seront utilisées pour représenter ces champignons dans le tableau (II).

Pour chaque essai, on opère de la manière suivante: un milieu nutritif constitué de pomme de terre, de glucose et de gelose (milieu PDA) est introduit en surfusion dans une série de boîtes de Petri (20 ml par boîte) après stérilisation à l'autoclave à 120°C.

Au cours du remplissage des boîtes, on injecte, dans le milieu en surfusion, une solution acétonique de la matière active, pour obtenir la concentration finale désirée.

On prend comme témoin des boîtes de Pétri analogues aux précédentes, dans lesquelles on coule des quantités similaires d'un milieu nutritif ne contenant pas de matière active.

Après 24 ou 48 h chaque boîte est ensemencée par dépôt d'un fragment de mycelium provenant d'une culture précédente du même champignon.

Les boîtes sont conservées pendant 2 à 10 jours (selon le champignon testé) à 22°C et on compare alors la croissance du champignon dans les boîtes contenant la matière active à tester, à celle du même champignon dans la boîte utilisée comme témoin.

On détermine ainsi pour chaque composé testé la plus faible dose permettant d'inhiber à 80-100 % le développement du champignon considéré. Cette dose est appelée "Concentration minimale inhibitrice".

Ces concentrations minimales inhibitrices, exprimées en ppm, sont rapportées dans le tableau suivant, dans lequel les abréviations ont la signification indiquée plus haut.

| COMPOSE | Dose minimale inhibitrice en mg/l | | | | | |
|---|---|---|---|---|---|---|
| | CERC | HELM G | HELM T | PYRE | SEPT N | BOT |
| 2 (A) | 10 | 30 | 10 | 100 | 30 | 30 |
| 1 | 3 | 100 | 10 | 10 | 30 | 30 |

Les concentrations de suspensions de matière active utilisées dans les exemples précédents ont été indiquées en g/l et correspondent sensiblement à des doses d'applications pourvues des mêmes chiffres mais exprimées en g/ha.

Les exemples suivants illustrent les propriétés des associations selon l'invention.

Exemple 14 - Test in vitro sur Septoria nodorum (composé/iprodione)

Dans une série de boîtes de Petri stériles, on introduit, en surfusion, un milieu nutritif.

Au cours du remplissage, on injecte dans le milieu en surfusion, une solution acétonique de la matière active constituée par l'association du composé obtenu selon l'exemple 1 avec l'iprodione dans des proportions variées pour obtenir la concentration désirée de matière active.

On prend comme témoins, des boîtes de Pétri analogues aux précédentes remplies comme indiqué ci-dessus sauf que le milieu nutritif ne contient pas de matière active.

Au bout de 24 heures, chaque boîte est ensemencée par dépôt d'un fragment de mycélium provenant d'une culture précédente du même champignon.

Les boîtes sont conservées 15 jours à 22 °C ± 2 et on compare la croissance du champignon dans les boîtes contenant la ou les matières actives à tester, à celle du même champignon dans les boîtes témoins.

Dans ces conditions, le pourcentage d'inhibition se calcule selon la formule suivante :

$$I = \frac{T - TT}{T - d} \times 100$$

dans laquelle :
I est le pourcentage d'inhibition,
T est le diamètre moyen, en mm, du développement mycélien dans la boîte témoin,
TT est le diamètre moyen, en mm, du développement mycélien dans la boîte traitée, et
d est le diamètre du fragment mycélien déposé au début de l'essai.

Dans ces conditions, on obtient les résultats consignés ci-dessous, sous forme des pourcentages d'inhibition réel $I_r$.

% d'inhibition sur Septoria nodorum

| IPRODIONE | Composé | | |
|---|---|---|---|
| Dose de matière active en mg/l | 0 | 0,3 | 3 |
| 0 | 0 | 43 | 69 |
| 3 | 70 | 85 | 95 |
| 10 | 83 | 90 | 100 |

Exemple 15 - Test in vitro sur Septoria nodorum (composé/fenpropimorphe)

mêmes conditions opératoires que dans l'exemple précédent.
Les résultats sont consignés ci-dessous

| FENPROPIMORPHE | Composé | | |
|---|---|---|---|
| Dose de matière active en mg/1 | 0 | 1 | 3 |
| 0 | 0 | 62 | 69 |
| 1 | 76 | 90 | 94 |
| 2 | 83 | 93 | 98 |

Exemple 16 - Test in vitro sur Septoria nodorum (composé/tridemorphe)

mêmes conditions opératoires que dans l'exemple précédent.
Les résultats sont consignés ci-dessous

| TRIDEMORPHE | Composé | | |
|---|---|---|---|
| Dose de matière active en mg/1 | 0 | 0,3 | 1 |
| 0 | 0 | 43 | 62 |
| 0,3 | 72 | 82 | 90 |
| 3 | 90 | 95 | 96 |

Exemple 17 - Test in vitro sur Septoria tritici (composé/captafol)

mêmes conditions opératoires que dans l'exemple précédent.
Les résultats sont consignés ci-dessous

| CAPTAFOL | Composé | |
|---|---|---|
| Dose de matière active en mg/1 | 0 | 3 |
| 0 | 0 | 35 |
| 100 | 84 | 92 |
| 300 | 100 | 100 |

Exemple 18 - Test in vitro sur Botrytis cinerea (composé/carbendazime)

mêmes conditions opératoires que dans l'exemple précédent.
Les résultats sont consignés ci-dessous

| CARBENDAZIME | Composé | |
|---|---|---|
| Dose de matière active en mg/1 | 0 | 10 |
| 0 | 0 | 35 |
| 0,08 | 72 | 85 |

Exemple 19 - Test in vitro sur Botrytis cinerea (composé/captane)

mêmes conditions opératoires que dans l'exemple précédent.
Les résultats sont consignés ci-dessous

| CAPTANE | Composé | |
|---|---|---|
| Dose de matière active en mg/1 | 0 | 10 |
| 0 | 0 | 35 |
| 10 | 9 | 86 |

Exemple 20 - Test in vitro sur Botrytis cinerea VE 35 (composé/iprodione)

(= résistant à l'iprodione et au carbendazime)mêmes conditions opératoires que dans l'exemple précédent.
Les résultats sont consignés ci-dessous

| IPRODIONE | Composé | |
|---|---|---|
| Dose de matière active en mg/l | 0 | 10 |
| 0 | 0 | 88 |
| 3 | | 90 |

Exemple 21 - Test in vitro sur Alternaria tenuis (composé/iprodione)

mêmes conditions opératoires que dans l'exemple précédent.
Les résultats sont consignés ci-dessous

| IPRODIONE | Composé | |
|---|---|---|
| Dose de matière active en mg/l | 0 | 10 |
| 0 | 0 | 68 |
| 10 | 90 | 100 |

Exemple 22 - Test in vitro sur Monilia lasca (composé/carbendazime)

mêmes conditions opératoires que dans l'exemple précédent.
Les résultats sont consignés ci-dessous

| CARBENDAZIME | Composé | | |
|---|---|---|---|
| Dose de matière active en mg/l | 0 | 0,3 | 1 |
| 0 | 0 | 31 | 77 |
| 0,008 | 76 | 86 | 100 |
| 0,015 | 92 | 92 | 95 |

Exemple 23 - Test in vitro sur Helminthosporum gramimeum (composé/fenpropimorphe)

mêmes conditions opératoires que dans l'exemple précédent.
Les résultats sont consignés ci-dessous

| FENPROPIMORPHE | Composé | | |
|---|---|---|---|
| Dose de matière active en mg/l | 0 | 10 | 30 |
| 0 | 0 | 65 | 75 |
| 0,1 | 0,00 | 78 | 69 |
| 0,3 | 0,00 | 52 | 75 |

Exemple 24 - Test in vitro sur Fusarium roseum (composé/chlorothalonil)

mêmes conditions opératoires que dans l'exemple précédent.
Les résultats sont consignés ci-dessous

| CHLOROTALONIL | Composé | | |
|---|---|---|---|
| Dose de matière active en mg/l | 0 | 0,3 | 30 |
| 0 | 0 | 56 | 49 |
| 100 | 51 | 51 | 75 |
| 300 | 73 | 74 | 83 |

Exemple 25 - Test in vitro sur Fusarium roseum (composé/manebe)

mêmes conditions opératoires que dans l'exemple précédent.
Les résultats sont consignés ci-dessous

| MANEBE | Composé | | |
|---|---|---|---|
| Dose de matière active en mg/l | 0 | 0,3 | 30 |
| 0 | 0 | 6 | 49 |
| 30 | 75 | 83 | 85 |
| 50 | 73 | 78 | 83 |

Exemple 26 - Test in vitro sur Venturia pirina (composé/captane)

mêmes conditions opératoires que dans l'exemple précédent.
Les résultats sont consignés ci-dessous

| CAPTANE | Composé | | |
|---|---|---|---|
| Dose de matière active en mg/l | 0 | 10 | 100 |
| 0 | 0 | 70 | 94 |
| 30 | 46 | 78 | 95 |
| 100 | 62 | 88 | 95 |

Exemple 27 - Test in vitro sur Pseudocercosporella herpotrichoïdes (composé/tridemorphe)

mêmes conditions opératoires que dans l'exemple précédent.
Les résultats sont consignés ci-dessous

| TRIDEMORPHE | Composé | | |
|---|---|---|---|
| Dose de matière active en mg/1 | 0 | 0,3 | 3 |
| 0 | 0 | 22 | 77 |
| 0,3 | 18 | 41 | 80 |
| 1 | 52 | 64 | 91 |
| 3 | 86 | 86 | 96 |

Exemple 28 - Test in vitro sur Erysiphe graminis forme spéciale hordei sur orge (oïdium de l'orge)

On opère de la même façon que pour l'exemple 11 avec comme matière active une association du composé obtenu selon l'exemple I avec le tridémorphe dans un rapport pondéral 1 : 1.

A la dose de 0,06 g/l protection, totale (supérieure à 95 %).

Exemple 29 - Test in vivo sur Botrytis cinerea sur tomate

On prépare une émulsion aqueuse de la même façon que celle indiquée à l'exemple 11 en utilisant comme matière active une association constituée du composé obtenu selon l'exemple I et d'iprodione dans un rapport pondéral égal à 1.

Des tomates cultivées en serre (variété Marmande) âgées de 30 à 40 jours sont traitées par pulvérisation avec l'émulsion aqueuse.

Après 24 ou 48 heures, les feuilles sont coupées et mises dans 2 boîtes de Pétri (diamètre 11 cm) dont le fond a été préalablement garni d'un disque de papier filtre humide (5 folioles par boîte).

L'inoculum est ensuite apporté à l'aide d'une seringue par dépôt de gouttes (3 gouttes par foliole) d'une suspension de spores. Cette suspension de spores de Botytris cinerea a été obtenue à partir d'une culture de 15 jours, mise ensuite en suspension dans une solution nutritive (80.000 unités/$cm^3$).

Le contrôle est fait 6 jours après la contamination par comparaison avec un témoin non traité.

A la dose de 0,66 g/l de matière active protection totale (supérieure à 95 %).

Exemple 30 - Test in vivo sur Erysiphe graminis f sp. hordei sur blé (oïdium du blé)

On opère de la même façon que pour l'exemple 11 avec comme matière active une association du composé obtenu selon l'exemple 1 avec le tridémorphe et le fenpropimorphe dans un rapport pondéral :
Composé de l'exemple 1 : tridémorphe ou fenpropimorphe = 1,1

Du blé, en godets, semé dans de la terre franche, est traité au stade de 10 cm de hauteur par pulvérisation d'une émulsion aqueuse (appelée bouillie) de concentration en matière active indiquée ci-après. L'essai est répété deux fois. Au bout de 24 heures, on saupoudre les plants de blé avec des spores d'Erysiphe graminis, le saupoudrage étant effectué à l'aide de plants malades.

La lecture se fait 8 à 12 jours après la contamination.

Dans ces conditions, on observe les résultats suivants :
A la dose de 0,063 g/l de matière active protection totale (supérieure ou égale à 95 %).

Exemple 31 - Test in vivo sur "Septoria Nodorum" agent de la septoriose du blé

On opère de la même façon que pour l'exemple 11 avec comme matière active une association du composé obtenu selon l'exemple 1 avec l'iprodione dans un rapport pondéral:composé de l'exemple 1 : iprodione = 1.

Du blé, en godets, semé dans de la terre franche, est traité au stade de 10 cm de hauteur par pulvérisation avec des émulsions aqueuses (appelées bouillies) de concentration en matière active indiquée ci-après. L'essai est répété deux fois.

Au bout de 24 heures, une suspension aqueuse de spores (150000 sp/$cm^3$) est pulvérisée sur le blé ;

cette suspension a été obtenue à partir de cultures de champignons in vitro. On place ensuite le blé pendant 8 jours en cellule d'incubation à environ 20°C et à 100 % d'humidité relative. Le contrôle de l'état des plants se fait entre le 8ème et le 15ème jour après la contamination par comparaison avec le témoin non traité.

Dans ces conditions, on observe à la dose de 0,25 g/l de matière active une protection supérieure à 90 %.

Exemple 32 - Test préventif in vivo sur Monilia sp et Penicillium sp. agents de la pourriture des fruits en particulier de la pomme.

Ces deux tests bien qu'étant indépendants ont été regroupés sous le même exemple car le mode opératoire est identique. On opère de la même façon que pour l'exemple 11 avec comme matière active une association du composé selon l'exemple 1, avec l'iprodione dans un rapport pondéral = 1.

Des pommes sont traitées par pulvérisation avec des suspensions aqueuses (appelées bouillies) de concentration en matière active indiquée ci-après. L'essai est répété deux fois.

Au bout de 24 heures, une suspension aqueuse de spores (50 000 sp/$cm^3$ dans le cas de Monilia sp et 250 000 sp/$cm^3$ dans le cas de Penicillium sp) est pulvérisée sur les pommes ; cette suspension a été obtenue à partir de fruits contaminés. On place ensuite les pommes en cellule d'incubation à environ 20°C et 90-100 % d'humidité relative. Le contrôle de l'état des fruits se fait entre les 5ème et 10ème jour après, la contamination par comparaison avec le témoin non traité.

Dans ces conditions, à la dose de 0,66 g/l on observe une protection de 49 % pour Monilia sp et 21 % pour Penicillium sp.

Exemple 33 - Test in vivo sur Erysiphe graminis f.sp hordei sur orge (oïdium de l'orge)

On opère de la même façon que pour l'exemple 11 avec comme matière active une association du composé obtenu selon l'exemple 1 avec le tridemorphe dans un rapport pondéral, composé de l'exemple 1 : tridemorphe = 0,033.

De l'orge, en godets, semé dans de la terre franche, et traitée au stade 10 cm de hauteur par pulvérisation d'une émulsion aqueuse (appelée bouillie) de concentration indiquée ci-après. L'essai est répété deux fois. Au bout de 24 heures, on saupoudre les plants d'orge avec des spores d'Erysiphe graminis, le saupoudrage étant effectué à l'aide de plants malades.

La lecture se fait 8 à 12 jours après la contamination.

Dans ces conditions, on observe les résultats suivants :

- à la dose de 0,1 g/l, 50 % de protection.

Exemple 34 - Test in vivo sur mildiou de la vigne (Plasmopora viticola)

On opère de la même façon que pour l'exemple 11 pour obtenir une émulsion aqueuse d'une matière active constituée du composé obtenu selon l'exemple 1 avec le Phosetyl A1 dans un rapport pondéral égal à 0,5.

Des boutures de vigne (Vitis vinifera), de variété Chardonnay, sont cultivées dans des godets. Lorsque ces plants sont âgés de 2 mois (stade 8 à 10 feuilles, hauteur 20 à 30 cm), ils sont traités par pulvérisation au moyen d'une suspension ou solution aqueuse de la matière à tester. Chaque plant de vigne reçoit environ 5 ml de la solution ou dispersion.

Après sèchage pendant 24 heures, on contamine chaque plant par pulvérisation au moyen d'une suspension aqueuse de spores de Plasmopora Viticola, responsable du mildiou de la vigne, à raison d'environ 1 ml/plant (soit environ $10^5$ spores par plant).

Après cette contamination, les plants de vigne sont mis en incubation pendant deux jours à 18°C environ, en atmosphère saturée d'humidité, puis pendant cinq jours à 20-22°C environ sous 90-100 % d'humidité relative.

La lecture se fait 7 jours après la contamination. Dans ces conditions, on observe les résultats suivants : - à 3 g/litre protection totale.

TABLEAU DE NOMENCLATURE

Chlorothalonil Tétrachloro-isophtalonitrile
Iprodione 3-(3,5-dichlorophényl)-N-isopropyl-2,4-dioxo imidazolidine-1-carboxamide
Fenpropimorphe (±)-cis-4-[3-(4-tert-butylphenyl)-2-methyl propyl]-2,6-diméthylmorpholine
Tridémorphe 2,6-diméthyl-4-tridécylmorpholine
Fenpropidine 1[3-(p-terbutylphenyl)2-méthyl propyl] piperidine
Dinocap 2-(1-méthylheptyl)-4,6-dinitrophényl crotonate
Dithianon 5,10-dihydro-5,10-dioxonaphto [2,3-b]-1,4-dithia-anthraquinone
Manèbe Ethylène bis (dithiocarbamate) de manganèse
Mancozèbe Complexe de manèbe avec un sel de zinc

Zinèbe Ethylène bis (dithiocarbamate) de zinc
Phoséthyl-Al Tris-0-éthylphosphonate d'aluminium
Captane N-(trichlorométhylthio) cyclohex-4-ene-1,2 dicarboximide
Carbendazime Méthyl benzimidazol-2-yl carbamate
Captafol N-(1,1,2,2-tétrachloroéthylthio) cyclohex-4-ene-1,2-dicarboximide
Cymoxanil 2-cyano-N-[(éthylamino) carbonyl]-2-(méthoxyimino) acétamide
Nuarimol α-(2-chlorophényl)-α-(4-fluoro -phényl)-5-pyrimidine méthanol
Diniconazol 1-(2,4-dichlorophenyl)-4,4-dimethyl-2-(1,2,4-triazol-1-yl)-1-penten-3-ol.

Ce dernier produit est décrit dans le brevet GB 2046260 déja mentionné.

**Revendications pour les états contractants: BE, CH, DE, FR, GB, IT, LI, LU, NL, SE**

1) Composés utilisables notamment comme fongicide, caractérisés en ce qu'ils ont pour formule

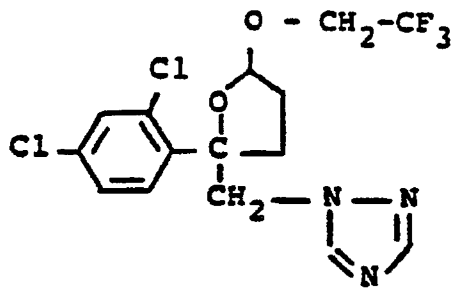

(I)

ainsi que les sels de ces produits.

2) Composé selon la revendication 1 caractérisé en ce qu'il est sous forme d'un mélange de diastéréoisomères.

3) Composé selon la revendication 1 caractérisé en ce qu'il est sous forme de diastéroisomère dans lequel les groupes triazolylméthyle et trifluoroéthoxy sont situés d'un même côté du plan du cycle tétrahydrofurannique.

4) Procédé de préparation de composés selon la revendication 1 caractérisé en ce qu'on fait réagir un composé de formule :

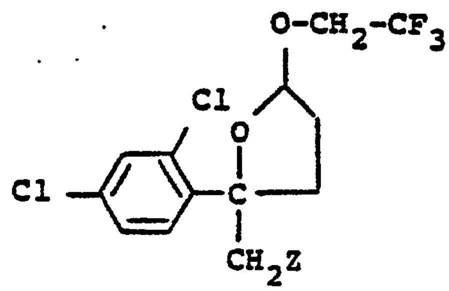

(II)

dans laquelle Z est un atome de chlore ou de brome, avec un dérivé alcalin d'un imidazole ou triazole.

5) Procédé selon la revendication 4 caractérisé en ce que la température est comprise entre 50 et 250°C, et/ou que le milieu contient un solvant polaire aprotique, et/ou que la concentration globale en réactifs est comprise entre 1 et 50 %.

6) Procédé de préparation de composés selon la revendication 1 caractérisé en ce qu'on fait agir du trifluoroéthanol $CF_3$-$CH_2OH$ en présence d'un catalyseur acide avec un composé de formule (IV)

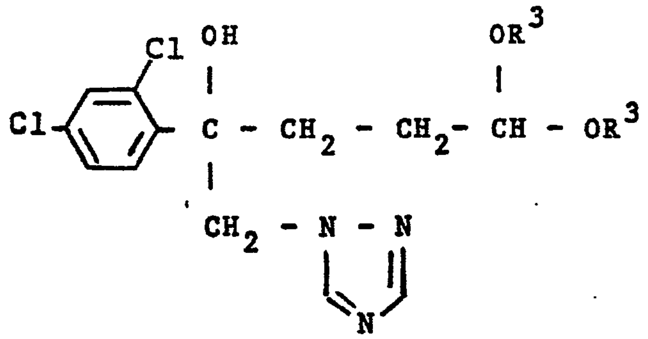

(IV)

dans laquelle $R^3$ est un radical organique, de préférence alkyle, deux radicaux $R^3$ pouvant constituer ensemble un radical unique divalent tel qu'alkylène.

7) Compositions fongicides, caractérisées en ce qu'elles contiennent comme matière active un produit selon l'une des revendications 1 à 3, cette matière active étant en association avec au moins un support inerte, acceptable en agriculture.

8) Compositions selon la revendication 7, caractérisées en ce qu'elles contiennent 0,5 à 95 % de matière active.

9) Compositions selon la revendication 8, caractérisées en ce qu'elles contiennent 1 à 95 % de support et 0,1 à 20 % d'agent tensioactif.

10)Procédé pour lutter contre les maladies fongiques des cultures, caractérisé en ce qu'on applique une dose efficace d'une matière active selon l'une des revendications 1 à 3.

11)Procédé selon la revendication 10, caractérisé en ce qu'on applique une composition selon l'une des revendications 7 à 9, la matière active étant appliquée à raison de 0,005 à 5 kg/ha, de préférence de 0,01 à 0,5 kg/ha.

12)Composé de formule (II)

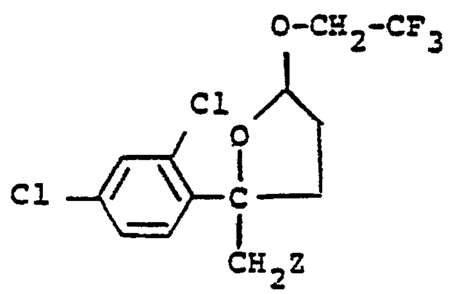

Z = Cl ou Br.

13)Associations utilisables notamment comme fongicide, caractérisées en ce qu'elles sont constituées d'un ou plusieurs composés selon les revendications 1 à 3 et d'un ou plusieurs composés choisis parmi le groupe (II) formé des sous classes suivantes :

1. Les dérivés chlorés ou nitrés du benzène comme le quintozène ou le chlorothalonil,
2. Les dérivés dicarboximides comme le captane, le folpel, le captafol, l'iprodione, la procymidone,
3. Les dérivés comprenant un ou plusieurs hétérocycles comme les quinoléines (éthoxyquine), les morpholines, (dodémorphe, tridémorphe, fenpropimorphe), les pipéridines (fenpropidine),
4. Les dérivés de l'acide phosphoreux comme les phosphites métalliques comme le phoséthyl-Al et l'acide phosphoreux lui-même.
5. Les dérivés de l'acide dithiocarbamique comme le manèbe, le mancozèbe ou le zinèbe,
6. Les dérivés du phénol comme le dinocap ou le binapacryl,
7. Les dérivés des quinones comme le dithianon, le chloranil,
8. Les dérivés de l'acide carbamique et des benzimidazoles comme le carbendazime, le bénomyl, le thiophanate-méthyl,
9. Les dérivé soufrés comme le dazomet ou l'étridiazole ou le soufre,
10. Les amines et les amides telles que le dichloran, la carboxine, la triforine, le cymoxanil, le métalaxyl, l'ofurace,
11. Les diazines telles que le chinométhionate, le fénarimol, l'anilazine, le nuarimol, le bupirimat, l'éthylrimol, le pyrazophe,
12. Les sulfamides telles que le dichlofluanide, le tolylfluanide,
13. Les guanidines telles que la doguadine.

14. Les triazoles tels que le diniconazole, le propiconazole, le triadimephon, le triadimenol, le diclobutazol, le bitertanol, le penconazol, le flutriafol.
15. Les imidazoles comme le prochloraz ou l'imazalil.
16. Le cuivre ou les dérivés du cuivre.

14)Associations selon la revendication 13) caractérisées en ce que les produits du groupe II sont choisis parmi les matières actives suivantes :
chlorothalonil, iprodione, fenpropimorphe, tridémorphe, fenpropidine, dinocap, dithianon, manèbe, mancozèbe, zinèbe, phoséthyl-Al, cymoxanil, captane, carbendazime, captafol, soufre, le cuivre et les dérivés du cuivre tels que l'oxychlorure de cuivre.

15)Associations selon l'une des revendications 13 ou 14) caractérisées en ce que le rapport pondéral du composé de formule (I) avec le ou les matières actives du groupe II est compris entre 0,0003 et 3000.

16)Associations selon la revendication 15) caractérisées en ce que le rapport pondéral est compris entre 0,001 et 1000.

17)Utilisation des associations selon l'une des revendications 13 à 16) à titre de fongicide.

18)Utilisation d'au moins un composé selon l'une des revendications 1 à 3 avec un ou plusieurs composés choisis parmi la fenpropidine, le fenpropimorphe, le tridemorphe, l'iprodione pour le traitement des maladies des céréales.

19)Utilisation d'au moins un composé selon l'une des revendications 1 à 3 avec un ou plusieurs composés choisis parmi le manèbe, le zinèbe, le mancozèbe, le soufre, le dinocap pour le traitement des vignes.

20)Utilisation d'au moins un composé selon l'une des revendications 1 à 3 avec un ou plusieurs composés choisis parmi le carbendazime, le captafol, le captane, le dithianon, le mancozèbe, le manèbe, le cuivre pour le traitement en arboriculture.

21)Composition fongicide caractérisée en ce qu'elle contient comme principe actif une association selon l'une des revendications 13 à 16) et au moins un support inerte, acceptable en agriculture.

22)Composition fongicide selon la revendication 21 caractérisée en ce qu'elle contient 0,5 à 95 % de principe actif.

23)Procédé pour lutter contre les maladies fongiques des cultures, caractérisé en ce qu'on applique une dose efficace d'une association selon l'une des revendications 13 à 16).

24)Procédé selon la revendication 23) caractérisé en ce qu'on applique l'association à raison de 0,005 à 5 kg/ha, de préférence de 0,01 à 5 kg/ha.

**Revendications pour les états contractants: AT, GR, ES**

1. Compositions fongicides caractérisées en ce qu'elles contiennent comme matière active un produit de formule

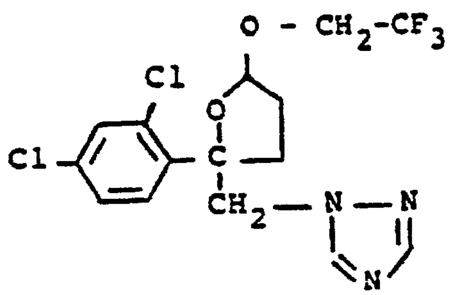

(I)

ou un de ses sels acceptables en agriculture, cette matière active étant en association avec au moins un support inerte, acceptable en agriculture.

2. Compositions selon la revendication 1, caractérisées en ce qu'elles contiennent 0,5 à 95% de matière active.

3. Compositions selon la revendication 2, caractérisées en ce qu'elles contiennent 1 à 95% de support et 0,1 à 20% d'agent tensioactif.

4. Compositions selon la revendication 1, caractérisées en ce que la matière active est sous forme diastéréoisomères.

5. Compositions selon la revendication 4, caractérisées en ce que la matière active est sous forme de diastéréoisomère dans lequel les groupes triazolylméthyle et trifluoroétoxy sont situés d'un même côté du plan du cycle tétrahydrofuranne.

6. Procédé pour lutter contre les maladies fongiques des cultures, caractérisé en ce qu'on applique une dose efficace d'une matière active de formule I ou un de ses sels acceptables en agriculture.

7. Procédé selon la revendication 6, caractérisé en ce qu'on applique une composition selon l'une des revendications 1 à 5, la matière active étant appliquée à raison de 0,005 à 5 kg/ha, de préférence de 0,01 à 0,5 kg/ha.

8. Procédé de préparation de composés de formule (I), caractérisé en ce qu'on fait réagir un composé de formule:

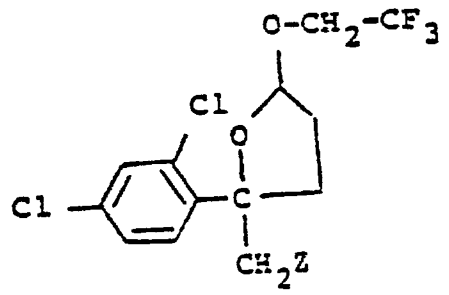

(II)

dans laquelle Z est un atome de chlore ou de brome, avec un dérivé alcalin d'un imidazole ou triazole.

9. Procédé selon la revendication 8, caractérisé en ce que la température est comprise entre 50 et 250°C, et/ou que le milieu contient un solvant polaire aprotique, et/ou que la concentration globale en réactifs est comprise entre 1 et 50%.

10. Procédé de préparation de composés selon la revendication 1, caractérisé en ce qu'on fait agir du trifluoroéthanol $CF_3–CH_2OH$ en présence d'un catalyseur acide avec un composé de formule (IV)

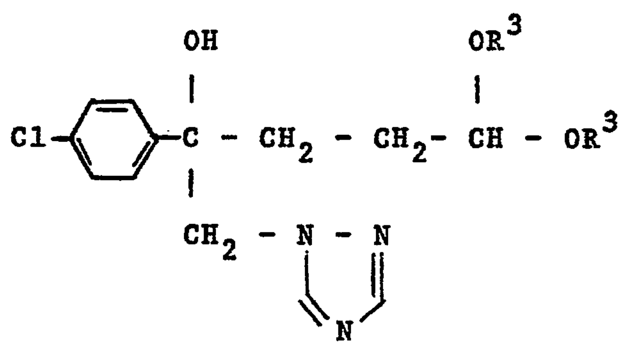

(IV)

dans laquelle $R^3$ est un radical organique, de préférence alkyle, deux radicaux $R^3$ pouvant constituer ensemble un radical unique divalent tel qu'alkylène.

11. Compositions fongicides caractérisées en ce qu'elles contiennent comme matière active l'association d'un ou plusieurs composés de formule (I) et d'un ou plusieurs composés choisis parmi le groupe (II) formé des sous-classes suivantes:

1. Les dérivés chlorés ou nitrés du benzène comme le quintozène ou le chlorothalonil,
2. Les dérivés dicarboximides comme le captane, le folpel, le captafol, l'iprodione, la procymidone,
3. Les dérivés comprenant un ou plusieurs hétérocycles comme les quinoléines (éthoxyquine), les morpholines, (dodémorphe, tridémorphe, fenpropimorphe), les pipéridines (fenpropidine),
4. Les dérivés de l'acide phosphoreux comme les phosphites métalliques comme le phoséthyl-Al et l'acide phosphoreux lui-même.
5. Les dérivés de l'acide dithiocarbamique comme le manèbe, le mancozèbe ou le zinèbe,
6. Les dérivés du phénol comme le dinocap ou le binapacryl,
7. Les dérivés des quinones comme le dithianon, le chloranil,
8. Les dérivés de l'acide carbamique et des benzimidazoles comme le carbendazime, le bénomyl, le thiophanate-méthyl,
9. Les dérivés soufrés comme le dazomet ou l'étridiazole ou le soufre,
10. Les amines et les amides telles que le dichloran, la carboxine, la triforine, le cymoxanil, le métalaxyl, l'ofurace,
11. Les diazines telles que le chinométhionate, le fénarimol, l'anilazine, le nuarimol, le bupirimat, l'éthylrimol, le pyrazophe,
12. Les sulfamides telles que le dichlofluanide, le tolylfluanide,
13. Les guanidines telles que la doguadine.
14. Les triazoles tels que le diniconazole, le propiconazole, la triadimephon, le triadimenol, le diclobutazol, le bitertanol, le penconazol, le flutriafol.
15. Les imidazoles comme le prochloraz ou l'imazalil.
16. Le cuivre ou les dérivés du cuivre.

12. Compositions selon la revendication 11, caractérisées en ce que les produits du groupe II sont choisis parmi les matières actives suivantes:

chlorothalonil, iprodione, fenpropimorphe, tridémorphe, fenpropidine, dinocap, dithianon, manèbe, mancozèbe, zinèbe, phoséthyl-Al, cymoxanil, captane, carbendazime, captafol, soufre, le cuivre et les dérivés du cuivre tels que l'oxychlorure de cuivre.

13. Compositions selon l'une des revendications 11 ou 12, caractérisées en ce que le rapport pondéral du composé de formule (I) avec le ou les matières actives du groupe II est compris entre 0,0003 et 3000.

14. Compositions selon la revendication 13, caractérisées en ce que le rapport pondéral est compris entre 0,001 et 3000.

15. Utilisation des associations selon l'une des revendications 11 à 14 à titre de fongicide.

16. Utilisation d'au moins un composé selon l'une des revendications 1 à 5 avec un ou plusieurs composés choisis parmi la fenpropidine, le fenpropimorphe, le tridemorphe, l'iprodione pour le traitement des maladies des céréales.

17. Utilisation d'au moins un composé selon l'une des revendications 1 à 5 avec un ou plusieurs composés choisis parmi le manèbe, le zinèbe, le mancozèbe, le soufre, le dinocap pour le traitement des vignes.

18. Utilisation d'au moins un composé selon l'une des revendications 1 à 5 avec un ou plusieurs composés choisis parmi le carbendazime, le captafol, le captane, le dithianon, le mancozèbe, le manèbe, le cuivre pour le traitement en arboriculture.

19. Procédé pour lutter contre les maladies fongiques des cultures, caractérisé en ce qu'on applique une dose efficace d'une des revendications 11 à 14.

20. Procédé selon la revendication 19, caractérisé en ce qu'on applique l'association à raison de 0,005 à 5 kg/ha, de préférence de 0,01 à 0,5 kg/ha.

**Claims for the contracting states: BE, CH, DE, FR, GB, IT, LI, LU, NL, SE**

1. Compounds capable of being used in particular as a fungicide, characterized in that their formula is

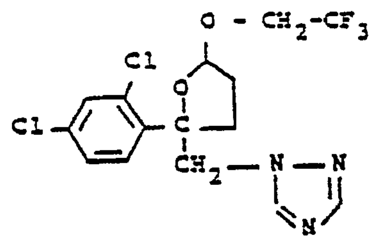

(I)

as well as the salts of these products.

2. Compound according to Claim 1, characterized in that it is in the form of a mixture of diastereoisomers.

3. Compound according to Claim 1, characterized in that it is in the form of a diastereoisomer in which the triazolylmethyl and trifluoroethoxy groups are situated on the same side of the plane of the tetrahydrofuran ring.

4. Process for the preparation of compounds according to Claim 1, characterized in that a compound of formula:

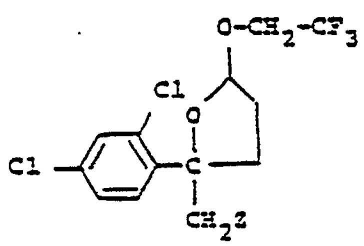

(II)

in which Z is a chlorine or bromine atom, is reacted with an alkali metal derivative of an imidazole or triazole.

5. Process according to Claim 4, characterized in that the temperature is between 50 and 250°C, and/or in that the medium contains an aprotic polar solvent, and/or in that the overall concentration of reactants is between 1 and 50%.

6. Process for the preparation of compounds according to Claim 1, characterized in that trifluoroethanol $CF_3-CH_2OH$ is reacted in the presence of an acidic catalyst with a compound of formula (IV)

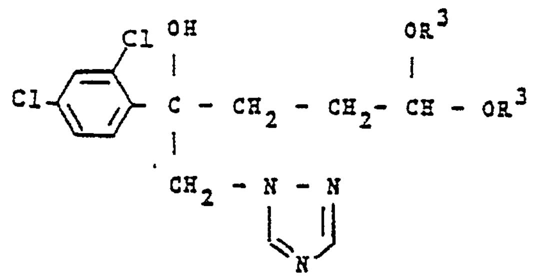

(IV)

in which $R^3$ is an organic radical, preferably alkyl, it being possible for two radicals $R^3$ to form together a single divalent radical such as alkylene.

7. Fungicidal compositions, characterized in that they contain as an active substance a product according to one of Claims 1 to 3, this active substance being in combination with at least one agriculturally acceptable inert carrier.

8. Compositions according to Claim 7, characterized in that they contain 0.5 to 95% of active substance.

9. Compositions according to Claim 8, characterized in that they contain 1 to 95% of carrier and 0.1 to 20% of surface-active agent.

10. Process for combating fungal diseases of crops, characterized in that an effective dosage of an active substance according to one of Claims 1 to 3 is applied.

11. Process according to Claim 10, characterized in that a composition according to one of Claims 7 to 9 is applied, the active substance being applied at a rate of 0.005 to 6 kg/ha, preferably from 0.01 to 0.5 kg/ha.

12. Compound of formula (II)

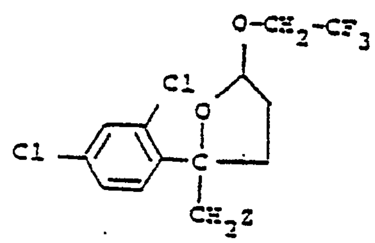

Z = Cl or Br.

13. Combinations capable of being used in particular as a fungicide, characterized in that they consist of one or more compounds according to one of Claims 1 to 3 and of one or more compounds chosen from the group (II) consisting of the following subclasses:

1. Chlorinated or nitrated benzene derivatives such as quintozene or chlorothalonil,
2. Dicarboxamide derivatives such as captan, folpel, captafol, iprodione and procymidone,
3. Derivatives containing one or more heterocyclic rings such as quinolines (ethoxyquine), morpholines (dodemorph, tridemorp, fenpropimorph) and piperidines (fenpropidine),
4. Phosphorous acid derivatives such as metal phosphites such as phosethyl-Al and phosphorous acid itself,
5. Dithiocarbamic acid derivatives such as maneb, mancozeb or zineb,
6. Phenol derivatives such as dinocap or binapacryl,
7. Quinone derivatives such as dithianon or chloroanil,
8. Carbamic acid and benzimidazole derivatives such as carbendazim, benomyl and thiophanate-methyl,
9. Sulphur derivatives such as dazomet or etridiazole or sulphur,
10. Amines and amides such as dichloran, carboxin, triforin, cymoxanil, metalaxyl and ofurace,
11. Diazines such as quinomethionate, fenarimol, anilazine, nuarimol, bupirimat, ethylrimol and pyrozophos,
12. Sulphamides such as dichlofluanide and tolyl-fluanide,
13. Guanidines such as doguadine,
14. Triazoles such as diniconazol, propiconazol, triadimephon, triadimenol, dichlobutazol, bitertanol, penconazol and flutriafol,
15. Imidazoles such as prochloraz or imazalil,
16. Copper or copper derivatives.

14. Combinations according to Claim 13, characterized in that the products of group II are chosen from the following active substances:

chlorothalonil, iprodione, fenpropimorph, tridemorph, fenpropidine, dinocap, dithianon, maneb, mancozeb, zineb, phosethyl-Al, cymoxanil, captan, carbendazim, captafol, sulphur, copper and copper derivatives such as copper oxychloride.

15. Combinations according to one of Claims 13 or 14, characterized in that the weight ratio of the compound of formula (I) to the active substance(s) of the group II is between 0.0003 and 3,000.

16. Combinations according to Claim 15, characterized in that the weight ratio is between 0.001 and 1,000.

17. Use of the combinations according to one of Claims 13 to 16 as a fungicide.

18. Use of at least one compound according to one of Claims 1 to 3 with one or more compounds chosen from fenpropidine, fenpropimorph, tridemorph or iprodione for the treatment of diseases of cereals.

19. Use of at least one compound according to one of Claims 1 to 3 with one or more compounds chosen from maneb, zineb, mancozeb, sulphur or dinocap for the treatment of vines.

20. Use of at least one compound according to one of Claims 1 to 3 with one or more compounds chosen from carbendazim, captafol, captan, dithianon, mancozeb, maneb and copper for treatment in arboriculture.

21. Fungicidal composition, characterized in that it contains as an active substance a combination according to one of Claims 13 to 16 and at least one agriculturally acceptable inert carrier.

22. Fungicidal composition according to Claim 21, characterized in that it contains 0.5 to 95% of active substance.

23. Process for combating fungal diseases of crops, characterized in that an effective dosage of a combination according to one of Claims 13 to 16 is applied.

24. Process according to Claim 23, characterized in that the combination is applied at a rate of 0.005 to 5 kg/ha, preferably from 0.01 to 5 kg/ha.

## Claims for the contracting states: AT, GR, ES

1. Fungicidal compositions, characterized in that they contain, as active substance, a product of formula

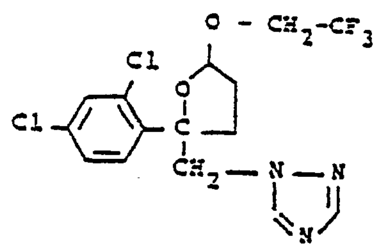

(I)

or one of its agriculturally acceptable salts, this active substance being in combination with at least one agriculturally acceptable inert carrier.

2. Compositions according to Claim 1, characterized in that they contain 0.5 to 95% of active substance.

3. Compositions according to Claim 2, characterized in that they contain 1 to 95% of carrier and 0.1 to 20% of surface-active agent.

4. Compositions according to Claim 1, characterized in that the active substance is in the form of diastereoisomers.

5. Compositions according to Claim 4, characterized in that the active substance is in the form of a diastereoisomer in which the triazolylmethyl nd trifluoroethoxy groups are situated on the same side of the plane of the tetrahydrofuran ring.

6. Process for combating fungal diseases of crops, characterized in that an effective dosage of an active substance of formula I or one of its agriculturally acceptable salts is applied.

7. Process according to Claim 6, characterized in that a composition according to one of Claims 1 to 5 is applied, the active substance being applied at a rate of 0.005 to 5 kg/ha, preferably from 0.01 to 0.5 kg/ha.

8. Process for the preparation of compounds of formula (I), characterized in that a compound of formula:

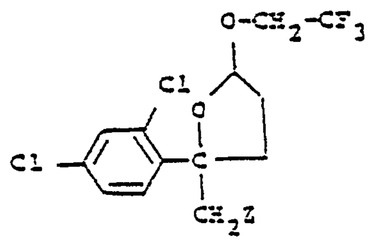

(II)

in which Z is a chlorine or bromine atom, is reacted with an alkali metal derivative of an imidazole or triazole.

9. Process according to Claim 8, characterized in that the temperature is between 50 and 250°C, and/or in that the medium contains an aprotic polar solvent, and/or in that the overall concentration of reactants is between 1 and 50%.

10. Process for the preparation of compounds according to Claim 1, characterized in that trifluoroethanol $CF_3-CH_2OH$ is reacted in the presence of an acidic catalyst with a compound of formula (IV)

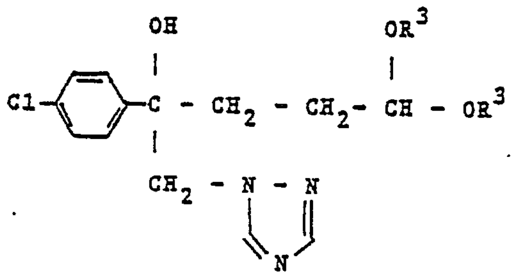

(IV)

in which $R^3$ is an organic radical, preferably alkyl, it being possible for two radicals $R^3$ to form together a single divalent radical such as alkylene.

11. Fungicidal compositions, characterized in that they contain, as active substance, the combination of one or more compounds of formula (I) and of one or more compounds chosen from the group (II) consisting of the following subclasses:

1. Chlorinated or nitrated benzene derivatives such as quintozene or chlorothalonil,
2. Dicarboximide derivatives such as captan, folpel, captafol, iprodione and procymidone,
3. Derivatives containing one or more heterocyclic rings such as quinolines (ethoxyquine), morpholines (dodemorph, tridemorph, fenpropimorph) and piperidines (fenpropidine),
4. Phosphorous acid derivatives such as metal phosphites such as phosethyl-Al and phosphorous acid itself,
5. Dithiocarbamic acid derivatives such as maneb, mancozeb or zineb,
6. Phenol derivatives such as dinocap or binapacryl,
7. Quinone derivatives such as dithianon or chloroanil,
8. Carbamic acid and benzimidazole derivatives such as carbendazim, benomyl and thiophanate-methyl,
9. Sulphur derivatives such as dazomet or etridiazole or sulphur,
10. Amines and amides such as dichloran, carboxin, triforin, cymoxanil, metalaxyl and ofurace,
11. Diazines such as quinomethionate, fenarimol, anilazine, nuarimol, bupirimat, ethylrimol and pyrazophos,
12. Sulphamides such as dichlofluanide and tolyl-fluanide,
13. Guanidines such as doguadine,
14. Triazoles such as diniconazol, propiconazol, triadimephon, triadimenol, dichlobutazol, bitertanol, penconazol and flutriafol,
15. Imidazoles such as prochloraz or imazalil,
16. Copper or copper derivatives.

12. Compositions according to Claim 11, characterized in that the products of the group II are chosen from the following active substances:
chlorothalonil, iprodione, fenpropimorph, tridemorph, fenpropidine, dinocap, dithianon, maneb, mancozeb, zineb, phosethyl-Al, cymoxanil, captan, carbendazim, captafol, sulphur, copper and copper derivatives such as copper oxychloride.

13. Compositions according to one of Claims 11 or 12, characterized in that the weight ratio of the compound of formula (I) to the active substance(s) of the group II is between 0.0003 and 3,000.

14. Compositions according to Claim 13, characterized in that the weight ratio is between 0.001 and 3,000.

15. Use of the combinations according to one of Claims 11 to 14 as a fungicide.

16. Use of at least one compound according to one of Claims 1 to 5 with one or more compounds chosen from fenpropidine, fenpropimorph, tridemorph or iprodione for the treatment of diseases of cereals.

17. Use of at least one compound according to one of Claims 1 to 5 with one or more compounds chosen from maneb, zineb, mancozeb, sulphur or dinocap for the treatment of vines.

18. Use of at least one compound according to one of Claims 1 to 5 with one or more compounds chosen from carbendazim, captafol, captan, dithianon, mancozeb, maneb and copper for treatment in arboriculture.

19. Process for combating fungal diseases of crops, characterized in that an effective dosage of a combination according to one of Claims 11 to 14 is applied.

20. Process according to Claim 19, characterized in that the combination is applied at a rate of 0.005 to 5 kg/ha, preferably from 0.01 to 0.5 kg/ha.

**Patentansprüche für die Vertragsstaaten: BE, CH, DE, FR, GB, IT, LI, LU, NL, SE**

1. Insbesondere als Fungizid verwendbare Verbindungen der Formel (I)

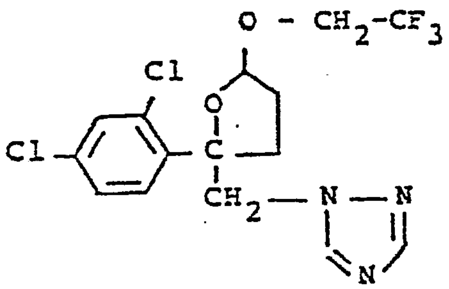

(I)

und ihre Salze.

2. Verbindungen nach Anspruch 1 in Form eines diastereoisomeren Gemisches.

3. Verbindungen nach Anspruch 1 in Form eines Diastereoisomeren, in dem die Triazolylmethyl- und Trifluorethoxygruppen auf der gleichen Seite der Tetrahydrofuranringebene sind.

4. Verfahren zur Herstellung der Verbindungen gemäß Anspruch 1, gekennzeichnet durch Umsetzung einer Verbindung der Formel (II)

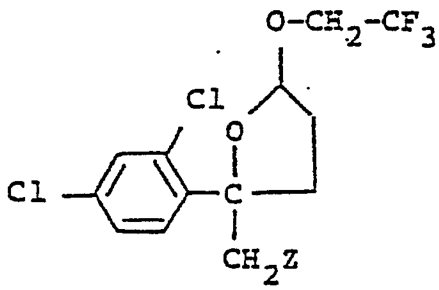

(II)

in der Z Chlor oder Brom ist, mit einem Alkaliderivat eines Imidazols oder Triazols.

5. Verfahren nach Anspruch 4, gekennzeichnet durch die Anwendung einer Temperatur von 50 bis 250°C und/oder eines ein polares aprotisches Lösungsmittel enthaltenden Mediums und/oder einer Gesamtkonzentration an reaktiven Bestandteilen von 1 bis 50%.

6. Verfahren zur Herstellung der Verbindungen gemäß Anspruch 1, gekennzeichnet durch Umsetzung von Trifluorethanol der Formel $CF_3-CH_2OH$ in Gegenwart eines sauren Katalysators mit einer Verbindung der Formel (IV)

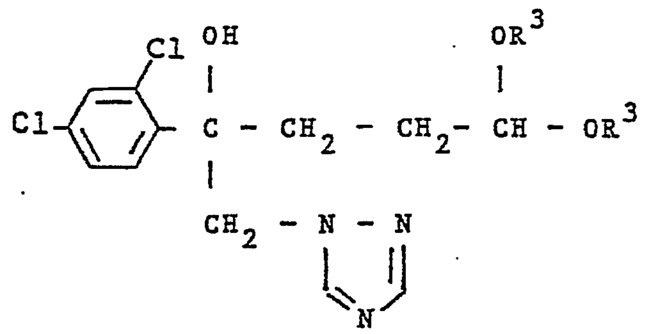

(IV)

in der $R^3$ ein organischer Rest und vorzugsweise Alkyl ist, wobei zwei Reste $R^3$ einen divalenten organischen Rest, wie Alkylen, bilden können.

7. Fungizide Mittel, gekennzeichnet durch eine Verbindung nach einem der Ansprüche 1 bis 3 als Wirkstoff in Kombination mit mindestens einem landwirtschaftlich zulässigen, inerten Träger.

8. Mittel nach Anspruch 7, gekennzeichnet durch einen Wirkstoffgehalt von 0,5 bis 95%.

9. Mittel nach Anspruch 8, gekennzeichnet durch einen Gehalt an Träger von 1 bis 95% und an grenzaktivem Mittel von 0,1 bis 20%.

10. Verfahren zur Bekämpfung von Pilzerkrankungen bei Kulturen, gekennzeichnet durch Aufbringen einer wirkungsvollen Dosis eines Wirkstoffs nach einem der Ansprüche 1 bis 3.

11. Verfahren nach Anspruch 10, gekennzeichnet durch Aufbringen eines fungiziden Mittels nach einem der Ansprüche 7 bis 9, wobei der Wirkstoff in einer Menge von 0,005 bis 5 kg/ha und vorzugsweise von 0,01 bis 0,5 kg/ha angewendet wird.

12. Verbindung der Formel (II)

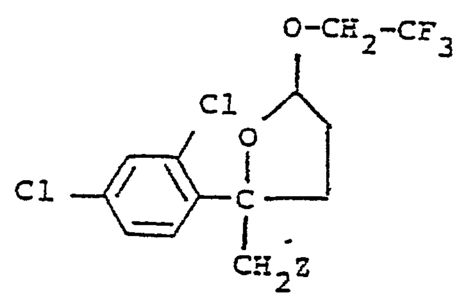

in der Z Chlor oder Brom ist.

13. Insbesondere als Fungizid verwendbare Mittel, gekennzeichnet durch mindestens eine Verbindung nach einem der Ansprüche 1 bis 3 und mindestens eine Verbindung, ausgewählt unter der Gruppe (II) der folgenden Unterklassen:

1. Chlor- oder Nitrobenzolderivate, wie Quintozen oder Chlorothalonil,
2. Dicarboximide, wie Captan, Folpel, Captafol, Iprodion oder Procymidon,
3. Derivate mit mindestens einem Heterocyclus, wie Chinoline (Ethoxyquin), Morpholine (Dodemorph, Tridemorph, Fenpropimorph) oder Piperidine (Fenpropidin),
4. Derivate der phosphorigen Säure, wie Metallphosphite (Phosethyl-Al), und phosphorige Säure selbst,
5. Dithiocarbamidsäure-Derivate, wie Maneb, Mancozeb oder Zineb,
6. Phenolderivate, wie Dinocap oder Binapacryl,
7. Chinonderivate, wie Dithianon oder Chloranil,
8. Carbamidsäure-Derivate und Benzimidazole, wie Carbendazim, Benomyl oder Thiophanat-methyl,
9. Schwefelderivate, wie Dazomet oder Etridiazol, oder Schwefel,
10. Amine und Amide wie Dichloran, Carboxin, Triforin, Cymoxanil, Metalaxyl oder Ofurace,
11. Diazine, wie Chinomethionat, Fenarimol, Anilazin, Nuarimol, Bupirimat, Ethylrimol oder Pyrazophos,
12. Sulfamide, wie Dichlofluanid oder Tolylfluanid,
13. Guanidine, wie Doguadin,
14. Triazole, wie Diniconazol, Propiconazol, Triadimephon, Triadimenol, Diclobutazol, Bitertanol, Penconazol oder Flutriafol,
15. Imidazole, wie Prochloraz oder Imazalil,
16. Kupfer und Kupferderivate.

14. Mittel nach Anspruch 13, dadurch gekennzeichnet, daß die Verbindungen der Gruppe (II) unter folgenden Wirkstoffen ausgewählt sind:
Chlorothalonil, Iprodion, Fenpropimorph, Tridemorph, Fenpropidin, Dinocap, Dithianon, Maneb, Macozeb, Zineb, Phosethyl-Al, Cymoxanil, Captan, Carbendazim, Captafol, Schwefel, Kupfer und Kupferderivate, wie Kupferoxychlorid.

15. Mittel nach Anspruch 13 oder 14, gekennzeichnet durch ein Masseverhältnis von Verbindungen der Formel (I) zu Wirkstoff(en) der Gruppe (II) von 0,0003 bis 3000.

16. Mittel nach Anspruch 15, gekennzeichnet durch ein Masseverhältnis von 0,001 bis 1000.

17. Verwendung der Mittel nach einem der Ansprüche 13 bis 16 als Fungizid.

18. Verwendung mindestens einer Verbindung gemäß einem der Ansprüche 1 bis 3 mit mindestens einer Verbindung, ausgewählt unter Fenpropidin, Fenpropimorph, Tridemorph oder Iprodion, zur Behandlung von Getreideerkrankungen.

19. Verwendung mindestens einer Verbindung gemäß einem der Ansprüche 1 bis 3 mit mindestens einer Verbindung, ausgewählt unter Maneb, Zineb, Mancozeb, Schwefel oder Dinocap, zur Behandlung von Weinstöcken.

20. Verwendung mindestens einer Verbindung gemäß einem der Ansprüche 1 bis 3 mit mindestens einer Verbindung, ausgewählt unter Carbendazim, Captafol, Captan, Dithianon, Mancozeb, Maneb oder Kupfer, zur Behandlung von Obstbäumen.

21. Fungizides Mittel, gekennzeichnet durch ein Mittel nach einem der Ansprüche 13 bis 16 als Wirkstoff und mindestens einem landwirtschaftlich zulässigen, inerten Träger.

22. Fungizides Mittel nach Anspruch 21, gekennzeichnet durch einen Wirkstoffgehalt von 0,5 bis 95%.

23. Verfahren zur Bekämpfung von Pilzerkrankungen bei Kulturen, gekennzeichnet durch Aufbringen einer wirkungsvollen Dosis eines Mittels nach einem der Ansprüche 13 bis 16.

24. Verfahren nach Anspruch 23, gekennzeichnet durch Aufbringen des Mittels in einer Menge von 0,005 bis 5 kg/ha und vorzugsweise von 0,1 bis 5 kg/ha.

**Patentansprüche für die Vertragsstaaten: AT, GR, ES**

1. Fungizides Mittel, gekennzeichnet durch eine Verbindung der Formel (I) oder eines ihrer landwirtschaftlich zulässigen Salze als Wirkstoff

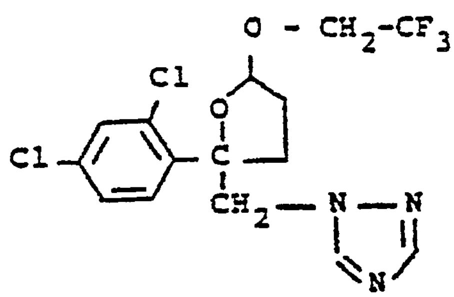

(I)

in Kombination mit mindestens einem landwirtschaftlich zulässigen inerten Träger.

2. Mittel nach Anspruch 1, gekennzeichnet durch einen Wirkstoffgehalt von 0,05 bis 95%.

3. Mittel nach Anspruch 2, gekennzeichnet durch einen Gehalt an Träger von 1 bis 95% und an grenzaktivem Mittel von 0,1 bis 20%.

4. Mittel nach Anspruch 1, dadurch gekennzeichnet, daß der Wirkstoff in Form eines Diastereoisomeren ist.

5. Mittel nach Anspruch 4, dadurch gekennzeichnet, daß der Wirkstoff in Form eines Diastereoisomeren ist, in dem die Triazolylmethyl- und Trifluorethoxygruppen auf der gleichen Seite der Tetrahydrofuranringebene sind.

6. Verfahren zur Bekämpfung von Pilzerkrankungen bei Kulturen, gekennzeichnet durch Aufbringen einer wirkungsvollen Dosis eines Wirkstoffs der Formel (I) oder eines seiner landwirtschaftlichen zulässigen Salze.

7. Verfahren nach Anspruch 6, gekennzeichnet durch Aufbringen eines Mittels nach einem der Ansprüche 1 bis 5, wobei der Wirkstoff in einer Menge von 0,005 bis 5 kg/ha und vorzugsweise von 0,01 bis 0,5 kg/ha angewendet wird.

8. Verfahren zur Herstellung der Verbindungen der Formel (I), gekennzeichnet durch Umsetzung einer Verbindung der Formel (II)

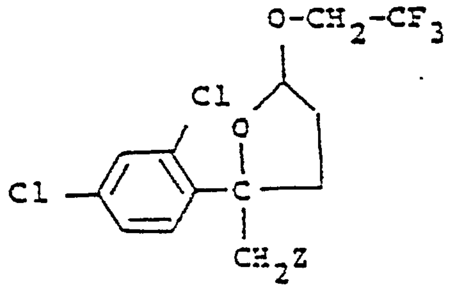

(II)

in der Z Chlor oder Brom ist, mit einem Alkaliderivat eines Imidazols oder Triazols.

9. Verfahren nach Anspruch 8, gekennzeichnet durch die Anwendung einer Temperatur von 50 bis 250°C und/oder eines ein polares aprotisches Lösungsmittel enthaltenden Mediums und/oder einer Gesamtkonzentration an reaktiven Bestandteilen von 1 bis 50%.

10. Verfahren zur Herstellung der Verbindungen gemäß Anspruch 1, gekennzeichnet durch Umsetzung von Trifluorethanol oder Formel $CF_3$–$CH_2OH$ in Gegenwart eines sauren Katalysators mit einer Verbindung der Formel (IV)

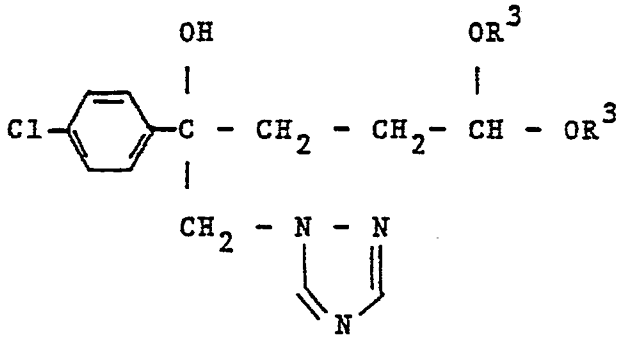

(IV)

in der $R^3$ ein organischer Rest und vorzugsweise Alkyl ist, wobei zwei Reste $R^3$ einen divalenten organischen Rest, wie Alkylen, bilden können.

11. Insbesondere als Fungizid verwendbare Mittel, gekennzeichnet durch als Wirkstoff mindestens einer Verbindung der Formel (I) und mindestens eine Verbindung, ausgewählt unter der Gruppe (II) der folgenden Unterklassen.

1. Chlor- oder Nitrobenzolderivate, wie Quintozen oder Chlorothalonil,
2. Dicarboximide, wie Captan, Folpel, Captafol, Iprodion oder Procymidon,
3. Derivate mit mindestens einem Heterocyclus, wie Chinoline (Ethoxyquin), Morpholine (Dodemorph, Tridemorph, Fenpropimorph) oder Piperidine (Fenpropidin),
4. Derivate der phosphorigen Säure, wie Metallphosphite (Phosethyl-Al), und phosphorige Säure selbst,
5. Dithiocarbamidsäure-Derivate, wie Maneb, Mancozeb oder Zineb,
6. Phenolderivate, wie Dinocap oder Binapacryl,
7. Chinonderivate, wie Dithianon oder Chloranil,
8. Carbamidsäure-Derivate und Benzimidazole, wie Carbendazim, Benomyl oder Thiophanat-methyl,
9. Schwefelderivate, wie Dazomet oder Etridiazol, oder Schwefel,
10. Amine und Amide wie Dichloran, Carboxin, Triforin, Cymoxanil, Metalaxyl oder Ofurace,
11. Diazine, wie Chinomethionat, Fenarimol, Anilazin, Nuarimol, Bupirimat, Ethylrimol oder Pyrazophos,
12. Sulfamide, wie Dichlofluanid oder Tolylfluanid,
13. Guanidine, wie Doguadin,
14. Triazole, wie Diniconazol, Propiconazol, Triadimephon, Triadimenol, Diclobutazol, Bitertanol, Penconazol oder Flutriafol,
15. Imidazole, wie Prochloraz oder Imazalil,
16. Kupfer und Kupferderivate.

12. Mittel nach Anspruch 11, dadurch gekennzeichnet, daß die Verbindungen der Gruppe (II) unter folgenden Wirkstoffen ausgewählt sind:
Chlorothalonil, Iprodion, Fenpropimorph, Tridemorph, Fenpropidin, Dinocap, Dithianon, Maneb, Mancozeb, Zineb, Phosethyl-Al, Cymoxanil, Captan, Carbendazim, Captafol, Schwefel, Kupfer und Kupferderivate, wie Kupferoxychlorid.

13. Mittel nach Anspruch 11 oder 12, gekennzeichnet durch ein Masseverhältnis von Verbindung der Formel (I) zu Wirkstoff(en) der Gruppe (II) von 0,0003 bis 3000.

14. Mittel nach Anspruch 13, gekennzeichnet durch ein Masseverhältnis von 0,001 bis 1000.

15. Verwendung der Mittel nach einem der Ansprüche 11 bis 14 als Fungizid.

16. Verwendung mindestens einer Verbindung gemäß einem der Ansprüche 1 bis 5 mit mindestens einer Verbindung, ausgewählt unter Fenpropidin, Fenpropimorph, Tridemorph oder Iprodion, zur Behandlung von Getreideerkrankungen.

17. Verwendung mindestens einer Verbindung gemäß einem der Ansprüche 1 bis 5 mit mindestens einer Verbindung, ausgewählt unter Maneb, Zineb, Mancozeb, Schwefel oder Dinocap, zur Behandlung von Weinstöcken.

18. Verwendung mindestens einer Verbindung gemäß einem der Ansprüche 1 bis 5 mit mindestens einer Verbindung, ausgewählt unter Carbendazim, Captafol, Captan, Dithianon, Mancozeb, Maneb oder Kupfer, zur Behandlung von Obstbäumen.

19. Verfahren zur Bekämpfung von Pilzerkrankungen bei Kulturen, gekennzeichnet durch Aufbringen einer wirkungsvollen Dosis eines Mittels nach einem der Ansprüche 11 bis 14.

20. Verfahren nach Anspruch 19, gekennzeichnet durch Aufbringen des Mittels in einer Menge von 0,005 bis 5 kg/ha und vorzugsweise von 0,1 bis 5 kg/ha.